# EUROPEAN PATENT SPECIFICATION

(11) **EP 3 394 589 B1**
(45) Date of publication and mention of the grant of the patent: **08.09.2021**
(21) Application number: 16880078.7
(22) Date of filing: 22.12.2016
(51) Int. Cl.: G01N 1/00, G01N 33/50, G01N 33/68

(54) **A METHOD TO IDENTIFY AN APPROACH FOR ACHIEVING MAMMALIAN FERTILIZATION AND TIME PERIOD FOR INSEMINATION**
VERFAHREN ZUR IDENTIFIZIERUNG EINES ANSATZES ZUR ERZIELUNG VON SÄUGETIERBEFRUCHTUNG UND ZEITRAUM ZUR BESAMUNG
PROCÉDÉ D'IDENTIFICATION D'UNE APPROCHE PERMETTANT DE RÉALISER UNE FÉCONDATION CHEZ LES MAMMIFÈRES ET D'IDENTIFICATION DE LA PÉRIODE D'INSÉMINATION

(30) Priority: 23.12.2015 US 201562387348 P; 15.01.2016 US 201662279315 P; 28.04.2016 US 201662328876 P; 28.04.2016 US 201662328885 P; 15.11.2016 US 201662422279 P
(43) Date of publication of application: 31.10.2018
(60) Divisional application: 21179036.5
(73) Proprietor: Androvia Life Sciences LLC, Mountainside, NJ 07092 (US)
(72) Inventor: TRAVIS, Alexander, J., Ithaca NY 14850 (US); CARDONA, Cristina, Highland Park NJ 08904 (US); MOODY, Melissa, A., Brick NJ 08724 (US); SIMPSON, Alana, J., Summit NJ 07901 (US); OSTERMEIER, G., Charles, Gillette NJ 07933 (US)
(74) Representative: Gill Jennings & Every LLP
(86) International application number: PCT/US2016/068204
(87) International publication number: WO 2017/112844

(56) References cited:
- WO-A2-2005/009222
- US-A- 5 994 086
- US-A1- 2010 248 302
- US-B1- 6 258 525
- Theodore Paniza ET AL: "A Bioassay to Measure Fertilization Competence of Human Spermatozoa Results Potential Clinical Application", , 2014, XP55598765, Retrieved from the Internet: URL:http://icsicenter.squarespace.com/s/Pa niza_ASRM_10_14_2014.pdf [retrieved on 2019-06-24]
- Aj Travis: "CAPACITATION DEFECTS ARE COMMON IN MEN QUESTIONING THEIR FERTILITYAND ARE INDEPENDENT OF STANDARD SEMEN ANALYSIS PARAMETERS.", , 18 October 2016 (2016-10-18), XP55618810, Retrieved from the Internet: URL:https://www.fertstert.org/article/S001 5-0282(16)62102-7/pdf [retrieved on 2019-09-05]
- MELISSA A. MOODY ET AL: "Validation of a laboratory-developed test of human sperm capacitation", MOLECULAR REPRODUCTION AND DEVELOPMENT, vol. 84, no. 5, 18 April 2017 (2017-04-18) , pages 408-422, XP055437335, NEW YORK, NY, US ISSN: 1040-452X, DOI: 10.1002/mrd.22801
- SELVARAJ ET AL.: 'GM1 Dynamics as a Marker for Membrane Changes Associated With the Process of Capacitation in Murine and Bovine Spermatozoa' JOURNAL OF ANDROLOGY vol. 28, no. 4, pages 588 - 599, XP055394967

## Description

### FIELD OF THE INVENTION

This invention relates generally to the field of male fertility and more specifically provides methods of identifying a reproductive approach for achieving mammalian fertilization.

### BACKGROUND

The diagnosis of male infertility is based predominantly on the results of standard semen analysis for concentration, total motility, progressive motility, volume, pH, viscosity and/or morphology. However, measurements of sperm morphology, motility and concentration do not assess fertilizing potential, including the complex changes that sperm undergo during residence within the female reproductive tract. In addition to the challenges associated with assessing fertilizing potential, cryopreservation is often used to preserve sperm cells and preserve male fertility for extended periods of time. Unfortunately, freezing and thawing can negatively affect sperm viability and function. Cryopreservation is reported to alter capacitation and shift/limit the fertilization window.

Newly developed fertility tests should determine the ability of sperm to fertilize, as well as initiate and maintain pregnancy (Oehninger et al., "Sperm functional tests," Fertil Steril. 102: 1528-33 (2014); Wang et al., "Limitations of semen analysis as a test of male fertility and anticipated needs from newer tests," Fertil Steril. 102: 1502-07 (2014)). While freshly ejaculated spermatozoa appear morphologically mature and motile, they are fertilization incompetent. They must first undergo a maturational process known as "capacitation," which renders them capable of fertilization (Austin, "The capacitation of the mammalian sperm," Nature, 170: 326 (1952); Chang, "Fertilizing capacity of spermatozoa deposited into the fallopian tubes," Nature, 168: 697-8 (1951)). In most species, capacitation is dependent upon the removal of sterols from the sperm plasma membrane (sterol efflux) and the influx of bicarbonate and calcium ions (Baldi et al., "Intracellular calcium accumulation and responsiveness to progesterone in capacitating human spermatozoa," J Androl. 12: 323-30 (1991); Bedu-Addo et al., "Bicarbonate and bovine serum albumin reversibly 'switch' capacitation-induced events in human spermatozoa," Mol Hum Reprod., 11: 683-91 (2005); Cohen et al., "Lipid modulation of calcium flux through Cav2.3 regulates acrosome exocytosis and fertilization," Dev Cell. 28: 310-21 (2014); Gadella et al., "Bicarbonate and its role in mammalian sperm function," Anim. Reprod. Sci. 82: 307-19 (2004)). The efflux of sterols that occurs during sperm capacitation changes membrane fluidity patterns and allows for the redistribution of specific membrane components (Cohen et al. 2014; Cross, "Role of cholesterol in sperm capacitation," Biol Reprod. 59: 7-11 (1998); Selvaraj et al., "Mechanisms underlying the micron-scale segregation of sterols and GM1 in live mammalian sperm," J Cell Physiol. 218: 522-36 (2007); Selvaraj et al, "GM1 dynamics as a marker for membrane changes associated with the process of capacitation in murine and bovine spermatozoa," J Androl. 28: 588-99 (2009))

Currently, there are few if any sensitive and simple capacitation biomarkers suitable for clinical application. For example, the phosphorylation of tyrosine residues has been well detailed in association with capacitation (Battistone et al., "Functional human sperm capacitation requires both bicarbonate-dependent PKA activation and down-regulation of Ser/Thr phosphatases by Src family kinases," Mol, Human Reprod. 19: 570-80 (2013); Osheroff et al., "Regulation of human sperm capacitation by a cholesterol efflux-stimulated signal transduction pathway leading to protein kinase A-mediated up-regulation of protein tyrosine phosphorylation," Mol, Human Reprod. 5: 1017-26 (1999); Visconti et al.. "Capacitation of mouse spermatozoa. I. Correlation between the capacitation state and protein tyrosine phosphorylation," Development, 121: 1129-37 (1995)). However, evaluating these events can take multiple days and provide only a semi-quantitative assessment, making it inappropriate for the clinical evaluation of male fertility.

It has been known for some time that sperm must undergo sterol efflux to become fertilization competent (Osheroff et al. 1999; Travis et al., "The role of cholesterol efflux in regulating the fertilization potential of mammalian spermatozoa," The Journal of Clinical Investigation, 110: 731-36 (2002)). In addition, cholesterol and other lipids, such as the ganglioside G_{M1}, are organized into microdomains within the sperm's plasma membrane (Asano et al., "Biochemical characterization of membrane fractions in murine sperm: Identification of three distinct sub-types of membrane rafts," J Cell Physiol., 218: 537-48 (2009); Asano et al., "Characterization of the proteomes associating with three distinct membrane raft sub-types in murine sperm," Proteomics, 10: 3494-505 (2010); Travis et al., "Expression and localization of caveolin-1, and the presence of membrane rafts, in mouse and Guinea pig spermatozoa," Dev Biol., 240: 599-610 (2001); Selvaraj et al. 2009). These membrane rafts consolidate signaling pathways, making them sensible candidates for mediating sperm function. Interestingly, predictable changes in G_{M1} localization patterns have been measured both in mouse and bull sperm that have been stimulated for capacitation (Selvaraj et al. 2007). What's more, G_{M1} regulates the activity of an R-type calcium channel, triggering a transient calcium flux that is essential for acrosome exocytosis and thus successful fertilization (Cohen et al. 2014). Paniza et al., "A bioassay to measure fertilization competence of human spermatozoa," Poster, 2014, noted that sperm capacitation was reflected in the pattern of GM1 localization. These findings substantiate the use of G_{M1} localization patterns to assess sperm function and accordingly male fertility.

Various G_{M1} localization patterns have been identified and associated with capacitation or non-capacitation. In particular, apical acrosome (AA) G_{M1} localization patterns and acrosomal plasma membrane (APM) G_{M1} localization patterns have been associated with capacitation in bovine and human sperm. Sperm capacitation can be quantitatively expressed as a Cap-Score™ value, generated via the Cap-Score™ Sperm Function Test ("Cap-Score™ Test" or "Cap-Score"), is defined as ([number of apical acrosome (AA) G_{M1} localization patterns + number of acrosomal plasma membrane (APM) G_{M1} localization patterns]/total number of G_{M1} labeled localization patterns) where the number of each localization pattern is measured and then ultimately converted to a percentage score. In addition to APM G_{M1} localization patterns and AA G_{M1} localization patterns, the other labeled localization patterns included Lined-Cell G_{M1} localization patterns, intermediate (INTER) G_{M1} localization patterns, post acrosomal plasma membrane (PAPM) G_{M1} localization patterns, apical acrosome/post acrosome (AA/PA) G_{M1} localization patterns, equatorial segment (ES) G_{M1} localization patterns, and diffuse (DIFF) G_{M1} localization patterns. (Travis et al., "Impacts of common semen handling methods on sperm function," The Journal of Urology, 195 (4), e909 (2016)).

### SUMMARY OF INVENTION

The invention provides a method for identifying an approach for achieving mammalian fertilization comprising the steps of: obtaining by imaging one or more to-fluorescence images from a first sample of *in vitro* capacitated sperm cells treated with a fluorescence label, wherein the sperm cells are displaying one or more G_{M1} localization patterns, wherein said to-fluorescence images are obtained at post *in vitro* capacitation times selected from: 0.1 hour to 5 hours; 0.1 hour to 8 hours; 0.1 to 12 hours; or 0.1 hour to 18 hours; measuring a number of apical acrosome (AA) G_{M1} localization patterns, a number of acrosomal plasma membrane (APM) G_{M1} localization patterns and a total number of G_{M1} localization patterns displayed in one or more t₀-fluorescence images to determine a percentage of to-[AA G_{M1} localization patterns plus APM G_{M1} localization patterns]; comparing the percentage of measured to-[AA G_{M1} localization patterns plus APM G_{M1} localization patterns] to a reference percentage of [AA G_{M1} localization patterns plus APM G_{M1} localization patterns] to determine a fertility status; and identifying a reproductive approach for achieving mammalian fertilization based on the determined fertility status, wherein a reference percentage of [AA G_{M1} localization patterns plus APM G_{M1} localization patterns] corresponding to: greater than 35 % indicates a high fertility status; one standard deviation below 35 % indicates a medium fertility status; and two or more standard deviations below 35 % indicates a low fertility status.

In an embodiment of the method of the invention, if the male has at least normal sperm concentration, then the reproductive approach for high fertility status is selected from the group consisting of: intercourse, intracervical insemination (ICI), pre-capacitating sperm prior to intracervical insemination, intrauterine insemination (IUI), or pre-capacitating sperm prior to intrauterine insemination,; the reproductive approach for medium fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; or the reproductive approach for low fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intrafallopian transfer, subzonal insemination (SUZI), or pre-capacitating sperm prior to subzonal insemination.

In a further embodiment of the method of the invention, if the male has a less than normal sperm concentration, then the reproductive approach for high fertility status is selected from group consisting of: intracervical insemination (ICI), pre-capacitating sperm prior to intracervical insemination, intrauterine insemination (IUI) or pre-capacitating sperm prior to intrauterine insemination; the reproductive approach for medium fertility status is selected from group consisting of: intracervical insemination (ICI), pre-capacitating sperm prior to intracervical insemination, intrauterine insemination (IUI) or pre-capacitating sperm prior to intrauterine insemination; in vitro fertilization (IVF), pre-capacitating sperm prior to in vitro fertilization; or the reproductive approach for low fertility status is selected from group consisting of: in vitro fertilization (IVF), pre-capacitating sperm prior to in vitro fertilization, intracytoplasmic sperm injection (ICSI), pre-capacitating sperm prior to intracytoplasmic sperm injection, gamete intra-fallopian transfer (GIFT), pre-capacitating sperm prior to gamete intrafallopian transfer, subzonal insemination (SUZI) or pre-capacitating sperm prior to subzonal insemination.

In a further embodiment, if the reproductive approach corresponds to pre-capacitating sperm prior to *in vitro* fertilization, intracytoplasmic sperm injection (ICSI), gamete intrafallopian transfer (GIFT), or subzonal insemination (SUZI), then the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

In an instance of each of the foregoing embodiments, the method may further comprise the steps of: obtaining by imaging one or more ti-fluorescence images from a second sample of *in vitro* capacitated sperm cells obtained from the same male as the first sample and treated with a fluorescence label displaying one or more G_{M1} localization patterns, wherein said ti-fluorescence images are obtained at post capacitation time ti, wherein ti is greater than to or greater than 18 hours; measuring a number of apical acrosome (AA) G_{M1} localization patterns, a number of acrosomal plasma membrane (APM) G_{M1} localization patterns and a total number of G_{M1} localization patterns displayed in one or more ti-fluorescence images to determine a percentage of ti-[AA G_{M1} localization patterns plus APM G_{M1} localization patterns]; comparing the percentage of ti-[AA G_{M1} localization patterns plus APM G_{M1} localization patterns] to the percentage of to-[AA G_{M1} localization patterns plus APM G_{M1} localization patterns] to determine an *in vivo* capacitation time, wherein a difference in the percentage of to-[AA G_{M1} localization patterns plus APM G_{M1} localization patterns] and the percentage of ti-[AA G_{M1} localization patterns plus APM G_{M1} localization patterns] of greater than one standard deviation from a standard of 35 % indicates an *in vivo* capacitation time greater than 12 hours; and a difference of less than one standard deviation from the standard of 35 % indicates an *in vivo* capacitation time of less than 12 hours; further if the *in vivo* capacitation time is determined to be greater than 12 hours, then a ti-fertility status is determined based on a comparison of the percentage of measured ti-[AA G_{M1} localization patterns plus APM G_{M1} localization patterns] to the reference percentage of [AA G_{M1} localization patterns plus APM G_{M1} localization patterns]; or, if the *in vivo* capacitation time is determined to be less than 12 hours, then a ti-fertility status is determined based on a comparison of the reference percentage of [AA G_{M1} localization patterns plus APM G_{M1} localization patterns] to the percentage of measured ti-[AA G_{M1} localization patterns plus APM G_{M1} localization patterns] or the percentage of measured to-[AA G_{M1} localization patterns plus APM G_{M1} localization patterns]; and based on the male's ti-fertility status and *in vivo* capacitation time, identifying a time period for insemination and a reproductive approach to use in order to achieve fertilization.

In an embodiment of the methods of the invention, the identifying step may also be based on one or more of the following: patient demographics, reproductive status of female partner, sperm concentration, total motility, progressive motility, semen volume, semen pH, semen viscosity and/or sperm morphology and combinations thereof.

In an embodiment of the methods of the invention, the more than one G_{M1} localization patterns may include AA G_{M1} localization pattern, APM G_{M1} localization pattern, Lined-Cell G_{M1} localization pattern, INTER G_{M1} localization pattern, PAPM G_{M1} localization pattern, AA/PA G_{M1} localization pattern, ES G_{M1} localization pattern, and DIFF G_{M1} localization pattern.

In an embodiment of the methods of the invention, the sperm cells may have been treated *in vitro* with capacitation conditions for a capacitation time period of: at least one hour; at least 3 hours; at least 12 hours; at least 18 hours; at least 24 hours; for a capacitation time period ranging between 0.5 hours to 3 hours; 3 hours to 12 hours; 6 hours to 12 hours; 3 hours to 24 hours; 12 hours to 24 hours; or 18 hours to 24 hours.

In an embodiment of the methods of the invention, the *in vitro* capacitated sperm cells may have been treated with a fixative for a fixative time period of: at least 0.5 hour; at least 3 hours; at least 12 hours; at least 18 hours; at least 24 hours; at least 30 hours; at least 36 hours; or at least 48 hours, for a fixation time period ranging between 0.5 hours to 3 hours; 3 hours to 12 hours; 6 hours to 12 hours; 3 hours to 18 hours; 6-18 hours; 6-24 hours; 12 hours to 24 hours; 18 hours to 24 hours; 18-30 hours; 18-36 hours; 24-30 hours; 24-26 hours; 18-48 hours; 24-48 hours; or 36-48 hours.

In an embodiment of the methods of the invention, the sperm cells may have been treated with cryopreservation procedures and stored prior to being treated *in vitro* with capacitation conditions.

### BRIEF DESCRIPTION OF THE DRAWINGS

The foregoing summary, as well as the following detailed description of the invention, will be better understood when read in conjunction with the appended drawings.

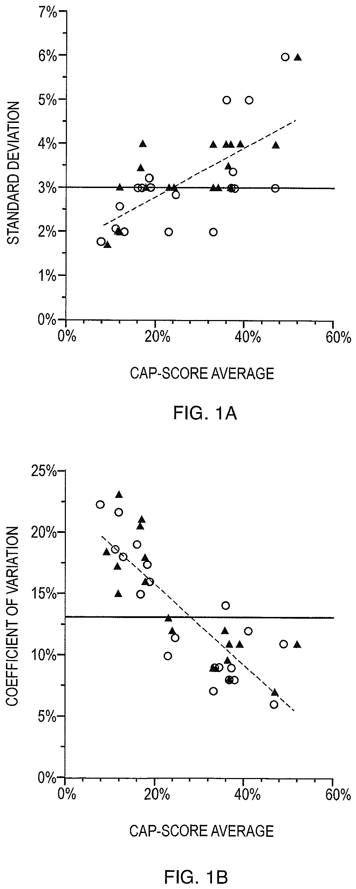

FIG. 1A illustrates a plot of the average Cap-Score is shown on the x-axis and the corresponding Standard Deviation is shown on the y-axis. The average SD for all images was found to be three (3) and is shown by the solid horizontal line. The dotted lines show the linear dependence of the SD (y = 0.06x + 0.02; r= 0.69; p=0.00).
FIG. 1B illustrates a plot of the average Cap-Score shown on the x-axis and Coefficient of Variation is shown on the y-axes. The CoV for all images was found to be thirteen (13) and is shown by the solid horizontal line. The dotted lines show the linear dependence of the CoV (y = -0.32x + 0.22; r=-0.84; p=0.00) to the Cap-Score average.

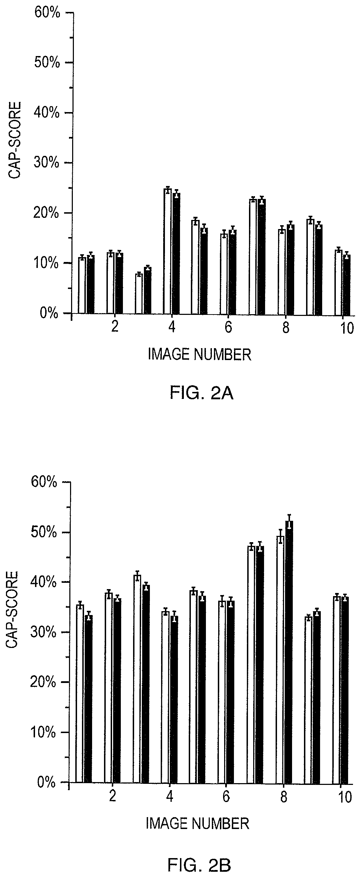

FIG. 2A illustrates the reproducibility of mean Cap-Scores between operators. Ten stitched images, containing up to 5,000 sperm each, were obtained for "less than normal" (more than one (1) SD below the mean Cap-Score result for a population of normal men. Two different readers determined Cap-Scores by randomly resampling each image 20 times and counting 150 cells each time (reader 1 open bars, reader 2 grey bars).
FIG. 2B illustrates the reproducibility of mean Cap-Scores between operators. Ten stitched images, containing up to 5,000 sperm each, were obtained for "presumed normal" classified as above one (1) SD below the mean Cap-Score result for a population of normal men. Two different readers determined Cap-Scores by randomly resampling each image 20 times and counting 150 cells each time (reader 1 open bars, reader 2 grey bars).

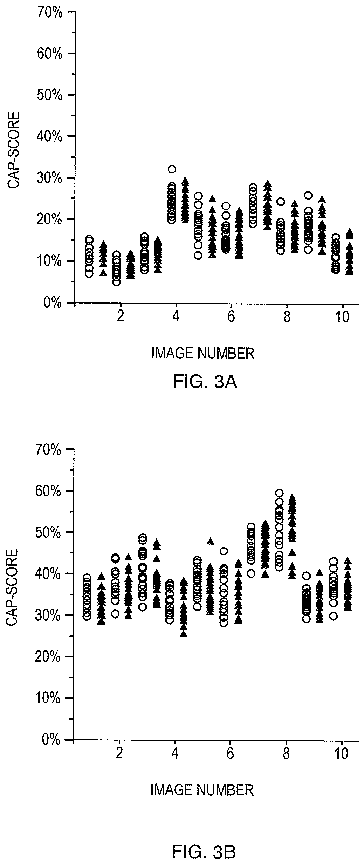

FIG. 3A illustrates the repeatability of Cap-Score variances between operators. Ten stitched images, containing up to 5,000 sperm each, were obtained for "less than normal" (more than one (1) SD below the mean Cap-Score result for a population of normal men. Two different readers determined Cap-Scores by randomly resampling each image 20 times and counting 150 cells each time (reader 1 open bars, reader 2 grey bars).
FIG. 3B illustrates the repeatability of Cap-Score variances between operators. Ten stitched images, containing up to 5,000 sperm each, were obtained for "presumed normal" classified as above one (1) SD below the mean Cap-Score result for a population of normal men. Two different readers determined Cap-Scores by randomly resampling each image 20 times and counting 150 cells each time (reader 1 open bars, reader 2 grey bars).

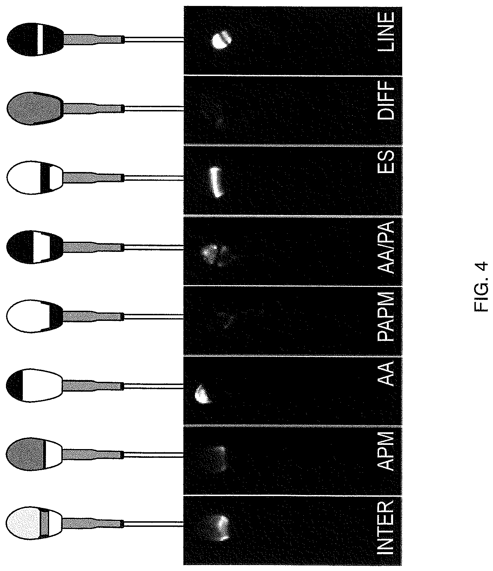

FIG. 4 illustrates various localization patterns of G_{M1} in normal human sperm and sperm from infertile males which form under in vitro capacitating conditions.

### DETAILED DESCRIPTION

As described herein, the time period for sperm capacitation among and within different males varies. It has been discovered that determining the time period for a male's sperm capacitation can be used to identify a time period for insemination and a reproductive approach to use during the insemination time period in order to achieve fertilization.

G_{M1} refers to monosialotetrahexosylganglioside and is a member of the ganglio series of gangliosides.

For human sperm, eight different G_{M1} localization patterns have been reported when the sperm was under in vitro capacitating conditions as illustrated in FIG. 4. To visualize the location patterns, the capacitated sperm were treated with labeling molecule for G_{M1}, such as cholera toxin b, which has a florescence detectable label on it.

INTER is characterized by a sperm cell where the vast majority of the fluorescence signal is in a band around the equatorial segment, with some signal in the plasma membrane overlying the acrosome. There is usually a gradient of the fluorescence signal, with the most at the equatorial segment and then progressively less toward the tip. There is often an increase in fluorescence signal intensity on the edges of the sperm head in the band across the equatorial segment.

Apical Acrosome "AA" is characterized by a sperm cell where the fluorescence signal is concentrated toward the apical tip, increased in brightness and reduced in area with signal.

Acrosomal Plasma Membrane "APM" is characterized by a sperm cell exhibiting a distributed fluorescence signal in the plasma membrane overlying the acrosome. APM signal is seen either from the bright equatorial INTER band moving apically toward the tip, or it can start further up toward the tip and be found in a smaller region, as it is a continuum with the AA.

Post-Acrosomal Plasma Membrane "PAPM' is characterized by a sperm cell where the fluorescence signal is exclusively in the post-acrosomal plasma membrane.

Apical Acrosome Post-Acrosome "AA/PA" is characterized by a sperm cell where the fluorescence signal is located both in the plasma membrane overlying the acrosome and post-acrosomal plasma membrane. The equatorial segment does not exhibit a fluorescence signal.

Equatorial Segment "ES" is characterized by a sperm cell having a bright fluorescence signal located solely in the equatorial segment. It may be accompanied by thickening of the sperm head across the equatorial region.

Diffuse "DIFF" is characterized by a sperm cell having a diffuse fluorescence signal located over the whole sperm head.

Lined-Cell is characterized by a sperm cell having a diffuse fluorescence signal ontop of the post-acrosomal region and at the plasma membrane overlying the acrosome as well as the bottom of the equatorial segment (i.e., the post acrosome/equatorial band). A fluorescence signal is missing around the equatorial segment.

The various G_{M1} localization patterns are identified by treating sperm cells with labeling molecule for G_{M1}, such as cholera toxin b, which has a florescence detectable label on it. The labeled sperm cells are then visualized using a fluorescence microscope as known to those of skill in the art.

Cap-Score is defined as the ratio of [the number of apical acrosome (AA) G_{M1} localization patterns + the number of acrosomal plasma membrane (APM) G_{M1} localization patterns] divided by [the total number of G_{M1} labeled localization patterns.] (Travis et al., "Impacts of common semen handling methods on sperm function," The Journal of Urology, 195 (4), e909 (2016)). To arrive at the number of different G_{M1} localization patterns, the number of, localization patterns, are counted for at least 100 sperm cells.

For the purposes of this application "to" corresponds to the number of hours after treating sperm cells with *in vitro* capacitation conditions and is selected from 0.1 hour to 5 hours; 0.1 hour to 8 hours; 0.1 to 12 hours; or 0.1 hour 18 hours.

For the purposes of this application "ti" corresponds to the number of hours after treating sperm cells with *in vitro* capacitation conditions and is greater than 18 hours or greater than to.

For the purposes of this application images is understood to mean (i) digital images; (ii) G_{M1} patterns directly viewed by an operator through an eye piece; or (iii) G_{M1} patterns discerned by flow cytometry.

For the purposes of this application "insemination" is understood to have a meaning dependent upon the reproductive approach. For example, for "intercourse," insemination is understood to mean introduction of sperm into a female's reproductive tract. For example, for "intracervical insemination (ICI)," insemination is understood to mean introduction of sperm into a female's cervix. For "intrauterine insemination (IUI)," insemination is understood to mean when sperm is introduced into a female's uterus. For *"in vitro* fertilization (IVF)," insemination is understood to mean when sperm are introduced into a droplet of medium containing egg cells (oocytes) to allow co-incubation of sperm and egg cell(s). For "pre-capacitating sperm prior to *in vitro* fertilization," insemination is understood to mean when sperm are introduced into a droplet of medium containing egg cells (oocytes) to allow co-incubation of sperm and egg cell(s). For "intracytoplasmic sperm injection (ICSI)," insemination is understood to mean injection of sperm or pre-capacitated sperm into an egg cell. For "gamete intra-fallopian transfer (GIFT)," insemination is understood to mean injection of sperm or pre-capacitated sperm and egg cell(s) into the female's Fallopian tubes. For "subzonal insemination (SUZI)," insemination is understood to mean injection of a single sperm cell or a single pre-capacitated sperm cell just beneath the zona pellucida. For purposes of this application, the term "cryopreservation" refers to the entire process of freezing, storing, and thawing the cells for use

As used herein below, the male is a mammal. In an embodiment, the male is a human. In another embodiment, the male is a non-human mammal. In one such embodiment, the male is a companion animal. In another embodiment, the male is an agricultural animal. In one such embodiment, the male is a canine, feline, equine, bovine, sheep, goat, pig, camellid, or buffalo.

In an embodiment, the disclosure provides for a method to identify an approach for achieving mammalian fertilization. A first sample, of *in vitro* capacitated sperm cells, is treated with a fluorescence label. One or more to-fluorescence images are obtained by imaging where the images display one or more G_{M1} localization patterns associated with to-fluorescence labeled *in vitro* capacitated sperm cells. The to-fluorescence images are obtained at post *in vitro* capacitation times selected from: 0.1 hour to 5 hours; 0.1 hour to 8 hours; 0.1 to 12 hours; or 0.1 hour 18 hours (to). A number of apical acrosome (AA) G_{M1} localization patterns, a number of acrosomal plasma membrane (APM) G_{M1} localization patterns and a total number of G_{M1} localization patterns are measured for the to-fluorescence labeled *in vitro* capacitated sperm cells displayed in one or more to-fluorescence images to determine a percentage of to-[AA G_{M1} localization patterns plus APM G_{M1} localization patterns]. A fertility status associated with a percentage of measured to-[AA G_{M1} localization patterns plus APM G_{M1} localization patterns] is determined wherein a reference percentage of [AA G_{M1} localization patterns plus APM G_{M1} localization patterns] corresponding to: greater than 35 % indicates a high fertility status; one standard deviation below 35 % indicates a medium fertility status; and two or more standard deviations below 35 % indicates a low fertility status. The percentage of measured to-[AA G_{M1} localization patterns and APM G_{M1} localization patterns] is compared to the reference percentage of [AA G_{M1} localization patterns plus APM G_{M1} localization patterns]. A reproductive approach is identified based on fertility status in order to achieve fertilization.

In an instance of the foregoing embodiment, wherein the male has at least normal sperm concentration: (i) the reproductive approach for high fertility status is selected from the group consisting of: intercourse, intracervical insemination (ICI), pre-capacitating sperm prior to intracervical insemination, intrauterine insemination (IUI), or pre-capacitating sperm prior to intrauterine insemination; (ii) the reproductive approach for medium fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; or (iii) the reproductive approach for low fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intrafallopian transfer, subzonal insemination (SUZI), or pre-capacitating sperm prior to subzonal insemination.

In another instance of the foregoing embodiment, wherein the male has a less than normal sperm concentration: (i) the reproductive approach for high fertility status is selected from group consisting of: intracervical insemination (ICI), pre-capacitating sperm prior to intracervical insemination, intrauterine insemination (IUI) or pre-capacitating sperm prior to intrauterine insemination; (ii) the reproductive approach for medium fertility status is selected from group consisting of: intracervical insemination (ICI), pre-capacitating sperm prior to intracervical insemination, intrauterine insemination (IUI) or pre-capacitating sperm prior to intrauterine insemination; *in vitro* fertilization (IVF), pre-capacitating sperm prior to *in vitro* fertilization, or (iii) the reproductive approach for low fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intrafallopian transfer, subzonal insemination (SUZI) or pre-capacitating sperm prior to subzonal insemination.

For the foregoing embodiments, where the sperm is pre-capacitated and the reproductive approach corresponds to pre-capacitating sperm prior to *in vitro* fertilization, the time period for pre-capacitation corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the sperm is pre-capacitated and the reproductive approach corresponds to intracytoplasmic sperm injection (ICSI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the sperm is pre-capacitated and the reproductive approach corresponds to gamete intra-fallopian transfer (GIFT), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the sperm is pre-capacitated and the reproductive approach corresponds to subzonal insemination (SUZI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

In an instance of each of the foregoing embodiments, a second sample of *in vitro* capacitated sperm cells is treated with a fluorescence label, wherein the second sample of *in vitro* capacitated sperm cells and first sample of *in vitro* capacitated sperm cells are associated with the same male. One or more ti-fluorescence images displaying one or more G_{M1} localization patterns associated with ti-fluorescence labeled *in vitro* capacitated sperm cells are obtained by imaging, wherein ti-fluorescence images are obtained at post capacitation time ti, wherein ti is selected from greater than to or greater than 18 hours. A number of apical acrosome (AA) G_{M1} localization patterns, a number of acrosomal plasma membrane (APM) G_{M1} localization patterns and a total number of G_{M1} localization patterns for the ti-fluorescence labeled *in vitro* capacitated sperm cells displayed in one or more ti-fluorescence images are measured to determine a percentage of ti-[AA G_{M1} localization patterns plus APM G_{M1} localization patterns]. The percentage of ti-[AA G_{M1} localization patterns plus APM G_{M1} localization patterns] to the percentage of to-[AA G_{M1} localization patterns plus APM G_{M1} localization patterns] is compared to determine an *in vivo* capacitation time selected from a late *in vivo* capacitation time greater than 12 hours or a standard *in vivo* capacitation time of less than 12 hours. A difference in percentage of to-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 % indicates a late *in vivo* capacitation time greater than 12 hours; or less than one standard deviation from the standard of 35 % indicates a standard *in vivo* capacitation time less than 12 hours. Further when a difference in percentage of to-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 % then a ti-fertility status is determined based on a comparison of the percentage of measured ti-[AA G_{M1} localization patterns plus APM G_{M1} localization patterns] to the reference percentage of [AA G_{M1} localization patterns plus APM G_{M1} localization patterns]; or less than one standard deviation from a standard of 35 % then a ti-fertility status is determined based on a comparison of the reference percentage of [AA G_{M1} localization patterns plus APM G_{M1} localization patterns] to the percentage of measured ti-[AA G_{M1} localization patterns plus APM G_{M1} localization patterns] or the percentage of measured to-[AA G_{M1} localization patterns plus APM G_{M1} localization patterns]. Based on the male's ti-fertility status and *in vivo* capacitation time, a time period for insemination and a reproductive approach to use are identified in order to achieve fertilization.

In an instance of the foregoing embodiment, wherein the male has at least normal sperm concentration and late *in vivo* capacitation time: (i) the reproductive approach for high ti-fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination, or pre-capacitating sperm prior to intrauterine insemination; (ii) the reproductive approach for medium ti-fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination; intrauterine insemination (IUI); pre-capacitating sperm prior to intrauterine insemination; or pre-capacitating sperm prior to *in vitro* fertilization, or (iii) the reproductive approach for low ti-fertility status is selected from group consisting of: modifying the timing of *in vitro* fertilization (IVF) to late *in vivo* capacitation time; modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time; pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In an instance of the foregoing embodiment, wherein the male has a less than normal sperm concentration and late *in vivo* capacitation time: (i) the reproductive approach for ti-high fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination, or pre-capacitating sperm prior to intrauterine insemination; (ii) the reproductive approach for ti-medium fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination, pre-capacitating sperm prior to intrauterine insemination; or pre-capacitating sperm prior to *in vitro* fertilization; or (iii) the reproductive approach for ti-low fertility status is selected from group consisting of: modifying the timing of *in vitro* fertilization (IVF) to late *in vivo* capacitation time; intracytoplasmic sperm injection (ICSI), modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time, pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In another instance of the foregoing embodiment, wherein the male has at least normal sperm concentration and standard *in vivo* capacitation time: (i) the reproductive approach for high ti-fertility status is selected from the group consisting of: intercourse, intracervical insemination (ICI), pre-capacitating sperm prior to intracervical insemination, intrauterine insemination (IUI), or pre-capacitating sperm prior to intrauterine insemination; (ii) the reproductive approach for medium ti-fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; or (iii) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intrafallopian transfer (GIFT), pre-capacitating sperm prior to gamete intra-fallopian transfer, subzonal insemination (SUZI), or pre-capacitating sperm prior to subzonal insemination.

In another instance of the foregoing embodiment, wherein the male has a less than normal sperm concentration and standard *in vivo* capacitation time: (i) the reproductive approach for high ti-fertility status is selected from group consisting of: intracervical insemination (ICI), pre-capacitating sperm prior to intracervical insemination, intrauterine insemination (IUI) or pre-capacitating sperm prior to intrauterine insemination; (ii) the reproductive approach for medium ti-fertility status is selected from group consisting of: intracervical insemination (ICI), pre-capacitating sperm prior to intracervical insemination, intrauterine insemination (IUI) or pre-capacitating sperm prior to intrauterine insemination; *in vitro* fertilization (IVF), pre-capacitating sperm prior to *in vitro* fertilization, or (iii) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI) or pre-capacitating sperm prior to subzonal insemination.

For the foregoing embodiments, where the sperm is pre-capacitated and the reproductive approach corresponds to pre-capacitating sperm prior to *in vitro* fertilization, the time period for pre-capacitation corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the sperm is pre-capacitated and the reproductive approach corresponds to intracytoplasmic sperm injection (ICSI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the sperm is pre-capacitated and the reproductive approach corresponds to gamete intra-fallopian transfer (GIFT), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

For embodiments where the sperm is pre-capacitated and the reproductive approach corresponds to subzonal insemination (SUZI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

In an instance of each of the foregoing embodiments, the identifying step is also based on one or more of the following: patient demographics, reproductive status of female partner, sperm concentration, total motility, progressive motility, semen volume, semen pH, semen viscosity and/or sperm morphology and combinations thereof.

In an instance of each of the foregoing embodiments, the more than one G_{M1} localization patterns include AA G_{M1} localization pattern, APM G_{M1} localization pattern, Lined-Cell G_{M1} localization pattern, INTER G_{M1} localization pattern, PAPM G_{M1} localization pattern, AA/PA G_{M1} localization pattern, ES G_{M1} localization pattern, and DIFF G_{M1} localization pattern.

In an instance of each of the foregoing embodiments, sperm cells are treated *in vitro* with capacitation conditions for a capacitation time period of: at least one hour; at least 3 hours; at least 12 hours; at least 18 hours; at least 24 hours; for a capacitation time period ranging between 0.5 hours to 3 hours; 3 hours to 12 hours; 6 hours to 12 hours; 3 hours to 24 hours; 12 hours to 24 hours; or 18 hours to 24 hours.

In an instance of each of the foregoing embodiments, the *in vitro* capacitated sperm cells are treated with a fixative for a fixative time period of: at least 0.5 hour; at least 3 hours; at least 12 hours; at least 18 hours; at least 24 hours; at least 30 hours; at least 36 hours; or at least 48 hours, for a fixation time period ranging between 0.5 hours to 3 hours; 3 hours to 12 hours; 6 hours to 12 hours; 3 hours to 18 hours; 6-18 hours; 6-24 hours; 12 hours to 24 hours; 18 hours to 24 hours; 18-30 hours; 18-36 hours; 24-30 hours; 24-26 hours; 18-48 hours; 24-48 hours; or 36-48 hours.

In an instance of each of the foregoing embodiments, the sperm cells were treated to cryopreservation procedures and stored prior to being treated *in vitro* with capacitation conditions.

The disclosure describes a further method to identify an approach for achieving mammalian fertilization. A sample of *to-in vitro* capacitated sperm cells is treated with a fluorescence label and a sample of *ti-in vitro* capacitated sperm cells is treated with a fluorescence label. One or more to-fluorescence images is obtained by imaging, the to-fluorescence images displaying one or more G_{M1} localization patterns associated with t₀-fluorescence labeled *in vitro* capacitated sperm cells. And one or more ti-fluorescence images is obtained, the ti-fluorescence displaying one or more G_{M1} localization patterns associated with ti-fluorescence labeled *in vitro* capacitated sperm cells. The to-fluorescence images are obtained at post *in vitro* capacitation times selected from: 0.1 hour to 5 hours; 0.1 hour to 8 hours; 0.1 to 12 hours; or 0.1 hour 18 hours (to); and the ti-fluorescence images being obtained at post capacitation time ti wherein ti is greater than to. A number of apical acrosome (AA) G_{M1} localization patterns, a number of acrosomal plasma membrane (APM) G_{M1} localization patterns and a total number of G_{M1} localization patterns are measured for the to-fluorescence labeled *in vitro* capacitated sperm cells displayed in the to-fluorescence images to determine a percentage of to-[AA G_{M1} localization patterns plus APM G_{M1} localization patterns]. And a number of apical acrosome (AA) G_{M1} localization patterns, a number of acrosomal plasma membrane (APM) G_{M1} localization patterns and a total number of G_{M1} localization patterns are measured for the ti-fluorescence labeled *in vitro* capacitated sperm cells displayed in the ti-fluorescence images to determine a percentage of ti-[AA G_{M1} localization patterns plus APM G_{M1} localization patterns]. The percentage of ti-[AA G_{M1} localization patterns plus APM G_{M1} localization patterns] is compared to the percentage of to-[AA G_{M1} localization patterns plus APM G_{M1} localization patterns] to determine an *in vivo* capacitation time selected from a late *in vivo* capacitation time greater than 12 hours or a standard *in vivo* capacitation time of 12 hours or less. A difference in percentage of to-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 % indicates a late *in vivo* capacitation time greater than 12 hours; or less than one standard deviation from a standard of 35 % indicates a standard *in vivo* capacitation time less than 12 hours. A reference percentage of [AA G_{M1} localization patterns plus APM G_{M1} localization patterns] corresponding to: greater than 35 % indicates a high fertility status; one standard deviation below 35 % indicates a medium fertility status; and two or more standard deviations below 35 % indicates a low fertility status. Further when a difference in percentage of to-[AA G_{M1} localization patterns plus APM G_{M1} localization patterns] and the percentage of ti-[AA G_{M1} localization patterns plus APM G_{M1} localization patterns] corresponding to: greater than one standard deviation from a standard of 35 %, then a ti-fertility status is determined based on a comparison of the percentage of measured ti-[AA G_{M1} localization patterns plus APM G_{M1} localization patterns] to the reference percentage of [AA G_{M1} localization patterns plus APM G_{M1} localization patterns]; or less than one standard deviation from a standard of 35 % then a ti-fertility status is determined based on a comparison of the reference percentage of [AA G_{M1} localization patterns plus APM G_{M1} localization patterns] to the percentage of measured ti-[AA G_{M1} localization patterns plus APM G_{M1} localization patterns] or the percentage of measured to-[AA G_{M1} localization patterns plus APM G_{M1} localization patterns]. Based on the male's ti-fertility status and *in vivo* capacitation time, a time period for insemination and a reproductive approach are identified to use in order to achieve fertilization.

In the preceding aspect of the disclosure the male has at least normal sperm concentration and late *in vivo* capacitation time: (i) the reproductive approach for high ti-fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination, or pre-capacitating sperm prior to intrauterine insemination; (ii) the reproductive approach for medium ti-fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination; intrauterine insemination (IUI); pre-capacitating sperm prior to intrauterine insemination; or pre-capacitating sperm prior to *in vitro* fertilization, or (iii) the reproductive approach for low ti-fertility status is selected from group consisting of: modifying the timing of *in vitro* fertilization (IVF) to late *in vivo* capacitation time; modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time; pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In the preceding aspect of the disclosure the male has a less than normal sperm concentration and late *in vivo* capacitation time: (i) the reproductive approach for ti-high fertility status is selected from the group consisting of: modifying the timing of intercourse to late *in vivo* capacitation time; modifying the timing of intracervical insemination (ICI) to late *in vivo* capacitation time; modifying the timing of intrauterine insemination (IUI) to late *in vivo* capacitation time; pre-capacitating sperm prior to intracervical insemination (ICI), or pre-capacitating sperm prior to intrauterine insemination (IUI); (ii) the reproductive approach for ti-medium fertility status is selected from group consisting of: modifying the timing of intracervical insemination (ICI) to late *in vivo* capacitation time; modifying the timing of intrauterine insemination (IUI) to late *in vivo* capacitation time; modifying the timing of *in vitro* fertilization (IVF) to late *in vivo* capacitation time; pre-capacitating sperm prior to intracervical insemination, pre-capacitating sperm prior to intrauterine insemination; or pre-capacitating sperm prior to *in vitro* fertilization; or (iii) the reproductive approach for ti-low fertility status is selected from group consisting of: modifying the timing of *in vitro* fertilization (IVF) to late *in vivo* capacitation time; intracytoplasmic sperm injection (ICSI), modifying the timing of intracytoplasmic sperm injection (ICSI) to late *in vivo* capacitation time; modifying the timing of gamete intra-fallopian transfer (GIFT) to late *in vivo* capacitation time; modifying the timing of subzonal insemination (SUZI) to late *in vivo* capacitation time, pre-capacitating sperm prior to *in vitro* fertilization, pre-capacitating sperm prior to intracytoplasmic sperm injection, pre-capacitating sperm prior to gamete intra-fallopian transfer, or pre-capacitating sperm prior to subzonal insemination.

In the preceding aspect of the disclosure the male has at least normal sperm concentration and standard *in vivo* capacitation time: (i) the reproductive approach for high ti-fertility status is selected from the group consisting of: intercourse, intracervical insemination (ICI), pre-capacitating sperm prior to intracervical insemination, intrauterine insemination (IUI), or pre-capacitating sperm prior to intrauterine insemination; (ii) the reproductive approach for medium ti-fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; or (iii) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI), or pre-capacitating sperm prior to subzonal insemination.

In the preceding aspect of the disclosure the male has a less than normal sperm concentration and standard *in vivo* capacitation time: (i) the reproductive approach for high ti-fertility status is selected from group consisting of: intracervical insemination (ICI), pre-capacitating sperm prior to intracervical insemination, intrauterine insemination (IUI) or pre-capacitating sperm prior to intrauterine insemination; (ii) the reproductive approach for medium ti-fertility status is selected from group consisting of: intracervical insemination (ICI), pre-capacitating sperm prior to intracervical insemination, intrauterine insemination (IUI) or pre-capacitating sperm prior to intrauterine insemination; *in vitro* fertilization (IVF), pre-capacitating sperm prior to *in vitro* fertilization, or (iii) the reproductive approach for low ti-fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI) or pre-capacitating sperm prior to subzonal insemination.

In each of the foregoing disclosure, the identifying step is also based on one or more of the following: patient demographics, reproductive status of female partner, sperm concentration, total motility, progressive motility, semen volume, semen pH, semen viscosity and/or sperm morphology and combinations thereof.

In each of the foregoing disclosure, the more than one G_{M1} localization patterns include AA G_{M1} localization pattern, APM G_{M1} localization pattern, Lined-Cell G_{M1} localization pattern, INTER G_{M1} localization pattern, PAPM G_{M1} localization pattern, AA/PA G_{M1} localization pattern, ES G_{M1} localization pattern, and DIFF G_{M1} localization pattern.

In each of the foregoing disclosure, the sperm cells are treated *in vitro* with capacitation conditions for a capacitation time period of: at least one hour; at least 3 hours; at least 12 hours; at least 18 hours; at least 24 hours; for a capacitation time period ranging between 0.5 hours to 3 hours; 3 hours to 12 hours; 6 hours to 12 hours; 3 hours to 24 hours; 12 hours to 24 hours; or 18 hours to 24 hours.

In each of the foregoing disclosure , the *in vitro* capacitated sperm cells are treated with a fixative for a fixative time period of: at least 0.5 hour; at least 3 hours; at least 12 hours; at least 18 hours; at least 24 hours; at least 30 hours; at least 36 hours; or at least 48 hours, for a fixation time period ranging between 0.5 hours to 3 hours; 3 hours to 12 hours; 6 hours to 12 hours; 3 hours to 18 hours; 6-18 hours; 6-24 hours; 12 hours to 24 hours; 18 hours to 24 hours; 18-30 hours; 18-36 hours; 24-30 hours; 24-26 hours; 18-48 hours; 24-48 hours; or 36-48 hours.

In each of the foregoing disclosure, the sperm cells were treated to cryopreservation procedures and stored prior to being treated *in vitro* with capacitation conditions.

The present disclosure provides for a method identifying an approach for achieving mammalian fertilization. *In vitro* capacitated sperm cells are treated with a fluorescence label. Data are generated that illustrate one or more G_{M1} localization patterns of the fluorescence label treated *in vitro* capacitated sperm cells said data obtained at post *in vitro* capacitation times selected from: 0.1 hour to 5 hours; 0.1 hour to 8 hours; 0.1 to 12 hours; or 0.1 hour 18 hours (to); and times greater than to (ti). A male's fertility status data are then characterized using the data of one or more G_{M1} localization patterns at those times. Based on the male's fertility status data, a time period for insemination and a reproductive approach are identified to use in order to achieve fertilization. The sperm cells may be cryopreserved and stored prior to being treated in vitro with capacitation conditions.

The sperm cells may be treated *in vitro* with capacitation conditions for a capacitation time period of: at least one hour; at least 3 hours; at least 12 hours; at least 18 hours; at least 24 hours; for a capacitation time period ranging between 0.5 hours to 3 hours; 3 hours to 12 hours; 6 hours to 12 hours; 3 hours to 24 hours; 12 hours to 24 hours; or 18 hours to 24 hours. Capacitation conditions include *in vitro* exposure to 2-hydroxypropyl-β-cyclodextrin. Non-capacitation conditions include lack of *in vitro* exposure to any of bicarbonate ions, calcium ions and a mediator of sterol efflux such as 2-hydroxypropyl-β-cyclodextrin for varying periods of time.

In one such other instance, the *in vitro* capacitated sperm cells are treated with a fixative for a fixation time period of: at least 0.5 hour; at least 3 hours; at least 12 hours; at least 18 hours; at least 24 hours; for a capacitation time period ranging between 0.5 hours to 3 hours; 3 hours to 12 hours; 6 hours to 12 hours; 3 hours to 24 hours; 12 hours to 24 hours; or 18 hours to 24 hours. The fixative may be paraformaldehyde or glutaraldehyde.

The male's fertility status data may be characterized by comparing data illustrating the G_{M1} localization patterns of *in vitro* capacitated sperm cells to reference data illustrating G_{M1} localization patterns of *in vitro* capacitated sperm cells for males having a known fertility status. The number of each G_{M1} labeled localization patterns is determined for a predetermined number of the in vitro capacitated sperm cells. A ratio is then calculated for a sum of the number of apical acrosome (AA) G_{M1} localization patterns and the number of acrosomal plasma membrane (APM) G_{M1} localization patterns over a sum of the total number of G_{M1} labeled localization patterns. The ratio of G_{M1} localization patterns is then compared to reference ratios of G_{M1} localization patterns for males having a known fertility status. The more than one G_{M1} localization patterns correspond to apical acrosome (AA) G_{M1} localization pattern, acrosomal plasma membrane (APM) G_{M1} localization pattern, Lined-Cell G_{M1} localization pattern, intermediate (INTER) G_{M1} localization pattern, post acrosomal plasma membrane (PAPM) G_{M1} localization pattern, apical acrosome/post acrosome (AA/PA) G_{M1} localization pattern, equatorial segment (ES) G_{M1} localization pattern, and diffuse (DIFF) G_{M1} localization pattern.

In another instance of the foregoing disclosure, the male's fertility status data are compared to data of known male fertility status which is associated with a known time period for insemination and associated with a known reproductive approach. The known fertility status includes: fertile with sperm capacitation within 3 hours; fertile with sperm capacitation within 12 hours, fertile with capacitation between 12 and 24 hours; and non-fertile.

In the foregoing disclosure, the reproductive approach may correspond to natural insemination approaches and artificial insemination approaches as known in the art. The reproductive approach may include: intercourse; intracervical insemination (ICI), intrauterine insemination (IUI), *in vitro* fertilization (IVF), intracytoplasmic sperm injection (ICSI), pre-capacitating sperm prior to *in vitro* fertilization, gamete intra-fallopian transfer (GIFT), and subzonal insemination (SUZI).

Where the reproductive approach corresponds to intercourse, the time period for intercourse is determined relative to the female's timing of ovulation, as visualized with ultrasonography, and/or predicted based on timing of the menstrual cycle, use of ovulation timing kits, changes in body temperature, or timing relative to one or more injections with one or more hormones designed to induce follicular growth and ovulation. For example, the insemination time period may correspond to: 96 hours before the time of ovulation; 72 hours before the time of ovulation; 48 hours before the time of ovulation; 24 hours before the time of ovulation; 12 hours before the time of ovulation; 6 hours before the time of ovulation; or at the time of ovulation.

Where the reproductive approach corresponds to ICI or IUI, the time period for insemination is determined relative to the female's timing of ovulation, as visualized with ultrasonography, and/or predicted based on timing of the menstrual cycle, use of ovulation timing kits, changes in body temperature, or timing relative to one or more injections with one or more hormones designed to induce follicular growth and ovulation. For example, the insemination time period may correspond to: 96 hours before the time of ovulation; 72 hours before the time of ovulation; 48 hours before the time of ovulation; 24 hours before the time of ovulation; 12 hours before the time of ovulation; 6 hours before the time of ovulation; or at the time of ovulation.

Where the reproductive approach corresponds to IVF, the time period for insemination corresponds to 3 hours before determination of pronuclear formation; 4 hours before determination of pronuclear formation; 6 hours before determination of pronuclear formation; 12 hours before determination of pronuclear formation; 18 hours before determination of pronuclear formation; 24 hours before determination of pronuclear formation; or 30 hours before determination of pronuclear formation.

Where the reproductive approach corresponds to pre-capacitating sperm prior to *in vitro* fertilization, the time period for pre-capacitation corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

Where the reproductive approach corresponds to intracytoplasmic sperm injection (ICSI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

Where the reproductive approach corresponds to gamete intra-fallopian transfer (GIFT), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

Where the reproductive approach corresponds to subzonal insemination (SUZI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

In some instances of the foregoing disclosure, other parameters may be used to identify a time period for insemination and a reproductive approach. The other parameters may include one or more of the following: patient demographics, reproductive status of female partner, sperm concentration, total motility, progressive motility, semen volume, semen pH, semen viscosity and/or sperm morphology and combinations thereof.

The present disclosure also provides a further method of identifying an approach for achieving mammalian fertilization. *In vitro* capacitated sperm cells are treated with a fluorescence label. One or more fluorescence images of fluorescence labeled in vitro capacitated sperm cells are obtained. A Cap-Score value is measured for fluorescence labeled *in vitro* capacitated sperm sample after the sperm cells are treated *in vitro* with capacitation conditions for varying periods of time. The Cap-Score value is compared to reference Cap-Score values associated with males of known fertility status at those times. Based on the Cap-Score value, a time period for insemination and a reproductive approach are identified for use in order to achieve fertilization.

The sperm cells are treated *in vitro* with capacitation conditions for a capacitation time period of: at least one hour; at least 3 hours; at least 12 hours; at least 18 hours; at least 24 hours; for a capacitation time period ranging between 0.5 hours to 3 hours; 3 hours to 12 hours; 6 hours to 12 hours; 3 hours to 24 hours; 12 hours to 24 hours; or 18 hours to 24 hours. Non-capacitation conditions include lack of *in vitro* exposure to any of bicarbonate ions, calcium ions and a mediator of sterol efflux such as 2-hydroxypropyl-β-cyclodextrin for varying periods of time.

The *in vitro* capacitated sperm cells are treated with a fixative for a time period of: at least 0.5 hour; at least 3 hours; at least 12 hours; at least 18 hours; at least 24 hours; at least 30 hours; at least 36 hours; or at least 48 hours, for a fixation time period ranging between 0.5 hours to 3 hours; 3 hours to 12 hours; 6 hours to 12 hours; 3 hours to 18 hours; 6-18 hours; 6-24 hours; 12 hours to 24 hours; 18 hours to 24 hours; 18-30 hours; 18-36 hours; 24-30 hours; 24-26 hours; 18-48 hours; 24-48 hours; or 36-48 hours. The fixative includes paraformaldehyde or glutaraldehyde.

In an instance of the foregoing disclosure, the Cap-Score value is compared to reference Cap-Score values for known male fertility status which is associated with known time period for insemination and associated with known reproductive approach. In such instances, the known fertility status includes: fertile with sperm capacitation within 3 hours; fertile with sperm capacitation within 12 hours, fertile with capacitation between 12 and 24 hours; and non-fertile.

In instances of the forgoing disclosure, Cap-Score corresponds to a ratio for a sum of a number of AA G_{M1} localization patterns and a number of APM G_{M1} localization patterns over a sum of a total number of G_{M1} labeled localization patterns, each determined for the *in vitro* capacitated sperm sample. The one or more G_{M1} labeled localization patterns comprises AA G_{M1} localization pattern, APM G_{M1} localization pattern, Lined-Cell G_{M1} localization pattern, INTER G_{M1} localization pattern, PAPM G_{M1} localization pattern, AA/PA G_{M1} localization pattern, ES G_{M1} localization pattern, and DIFF G_{M1} localization pattern.

The reproductive approach may correspond to natural insemination approaches and artificial insemination approaches as known in the art. The reproductive approach includes: intercourse; intracervical insemination (ICI), intrauterine insemination (IUI), *in vitro* fertilization (IVF), intracytoplasmic sperm injection (ICSI), pre-capacitating sperm prior to *in vitro* fertilization, gamete intra-fallopian transfer (GIFT), and subzonal insemination (SUZI).

Where the reproductive approach corresponds to intercourse, the time period for intercourse is determined relative to the female's timing of ovulation, as visualized with ultrasonography, and/or predicted based on timing of the menstrual cycle, use of ovulation timing kits, changes in body temperature, or timing relative to one or more injections with one or more hormones designed to induce follicular growth and ovulation. For example, the insemination time period may correspond to: 96 hours before the time of ovulation; 72 hours before the time of ovulation; 48 hours before the time of ovulation; 24 hours before the time of ovulation; 12 hours before the time of ovulation; 6 hours before the time of ovulation; or at the time of ovulation.

Where the reproductive approach corresponds to ICI or IUI, the time period for insemination is determined relative to the female's timing of ovulation, as visualized with ultrasonography, and/or predicted based on timing of the menstrual cycle, use of ovulation timing kits, changes in body temperature, or timing relative to one or more injections with one or more hormones designed to induce follicular growth and ovulation. For example, the insemination time period may correspond to: 96 hours before the time of ovulation; 72 hours before the time of ovulation; 48 hours before the time of ovulation; 24 hours before the time of ovulation; 12 hours before the time of ovulation; 6 hours before the time of ovulation; or at the time of ovulation.

Where the reproductive approach corresponds to IVF, the time period for insemination corresponds to 3 hours before determination of pronuclear formation; 4 hours before determination of pronuclear formation; 6 hours before determination of pronuclear formation; 12 hours before determination of pronuclear formation; 18 hours before determination of pronuclear formation; 24 hours before determination of pronuclear formation; or 30 hours before determination of pronuclear formation.

Where the reproductive approach corresponds to pre-capacitating sperm prior to *in vitro* fertilization, the time period for pre-capacitation corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

Where the reproductive approach corresponds to intracytoplasmic sperm injection (ICSI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

Where the reproductive approach corresponds to gamete intra-fallopian transfer ("GIFT"), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

Where the reproductive approach corresponds to subzonal insemination (SUZI), the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

The present disclosure also provides for a method of identifying an appropriate mechanism for achieving successful mammalian pregnancy. *In vitro* capacitated sperm cells are treated with a fluorescence label. One or more fluorescence images of fluorescence labeled in vitro capacitated sperm cells are obtained. A Cap-Score value is measured for fluorescence labeled *in vitro* capacitated sperm sample after the sperm cells are treated *in vitro* with capacitation conditions for varying periods of time. The Cap-Score value is compared to a reference Cap-Score value associated with fertile males. An appropriate mechanism to achieve a successful pregnancy is determined based on the Cap-Score value. The determination is also based on one or more of the following: patient demographics, reproductive status of female partner sperm concentration, total motility, progressive motility, semen volume, semen pH, semen viscosity and/or sperm morphology.

In an embodiment, a semen sample is processed using a wide orifice pipette having an orifice of sufficient size in diameter to prevent shearing of a sperm membrane and the semen sample is processed without use of a reagent that can damage sperm membranes. In one such embodiment, the processed semen sample is exposed to capacitating media, fixative, and reagents for determining G_{M1} localization patterns.

In an embodiment, a reagent that can damage sperm membranes is selected from the group consisting of: (i) a protease; (ii) a nuclease (iii) a mucolytic agent; (iv) a lipase; (v) an esterase and (vi) Glycoside hydrolases. Examples of compounds which may similarly interfere with the ability of sperm to respond to capacitation stimuli include: (i) a protease, including but not limited to, chymotrypsin, trypsin, collagenase, bromelain; (ii) a nucleases, including but not limited to, Dornase, HindIII, EcoRI; (iii) a mucolytic agent, including but not limited to, Erdostein, Acetylcysteine, Guiafenesin; (iv) a lipase, including but not limited to, Phospholipase Al, Phospholipase C, Lipoprotein lipase; (v) an esterase, including but not limited to, Cholinesterase, Thioesterase, Alkaline phosphatase; and (vi) Glycoside hydrolases, including but not limited to, Alpha-amylase, beta-galactosidase, hyaluronidase, neuorminodases, and lysozyme.

In instances of the foregoing disclosure, the population size for one or more G_{M1} labeled localization patterns is determined, such that the percent change about Cap-Score is minimized within an individual. The one or more G_{M1} labeled localization patterns comprises AA G_{M1} localization pattern, APM G_{M1} localization pattern, Lined-Cell G_{M1} localization pattern, intermediate (INTER) G_{M1} localization pattern, post acrosomal plasma membrane (PAPM) G_{M1} localization pattern, apical acrosome/post acrosome (AA/PA) G_{M1} localization pattern, equatorial segment (ES) G_{M1} localization pattern, and diffuse (DIFF) G_{M1} localization pattern.

### EXAMPLES

The following examples further describe and demonstrate illustrative embodiments of the present invention and instances of the disclosure.

### Example 1

### Sperm Handling Methods

Three common methods to reduce viscosity were evaluated. Ejaculates were: 1) Incubated for 0.25, 1.25 or 2 hours, 2) diluted 1:1 with Modified Human Tubal Fluid (Irvine Scientific; Santa Anna, CA) and then passed through a wide orifice transfer pipette ("WOTP") or a Pasteur pipette ("PP"), 3) Enzymatically digested with chymotrypsin ("chymo"). Pilot studies revealed that passage through a hypodermic needle negatively affected motility and membrane integrity and was not studied further. After liquefaction, samples were washed and incubated under capacitating (CAP) and non-capacitating (NC) conditions. Cap-Score values were obtained via fluorescence microscopy according to the calculation described above.

### Reliability and Reproducibility of Cap-Score™

Following liquefaction of semen samples from consenting men, sperm were washed, incubated, fixed and then evaluated via fluorescence microscopy for G_{M1} localization patterns. Precision of scoring within one sample, and variation between readers scoring the same samples were both assessed. Student's t-Test employing unequal variance was done using Microsoft Excel (2013).

### Cap-Score Correlation with Traditional Semen Analysis Parameters

Semen samples from consenting patients were liquefied, washed and incubated under both non-capacitating and capacitating conditions. Semen analysis was performed according to WHO guidelines. Cap-Score values were obtained via fluorescence microscopy. Statistical analyses were done using Microsoft Excel (2013) and XLSTAT (2015).

### Assessment of Capacitation using Cap-Score™ For Males of Demonstrated Fertility Compared to Males Being Assessed for Fertility

The Cap-Score values were determined on consenting men from two cohorts: 1) known fertility (pregnant partner or fathering a child less than 3 years old), and 2) patients seeking their first semen analysis. Following liquefaction, sperm were washed and 3 million incubated for 3 hour under non-capacitating (NC) and capacitating (CAP) conditions. Sperm were fixed overnight and G_{M1} localization patterns assessed via fluorescence microscopy.

### RESULTS

Liquefaction time, dilution and pipetting did not alter Cap-Score values. Control (incubation only), WOTP and PP treated samples had Cap-Score values of 41±4, 40±5, and 41±6 (n=5; CAP). A decrease in response to capacitating stimuli was observed when samples were liquefied using chymo (P=0.03). Control samples had Cap-Score values of 40±6 (n=5; CAP) whereas samples enzymatically liquefied had Cap-Score values of 31±4 (n=5; CAP). Because chymo is a protease that can cut membrane proteins, it was examined to determine if the reduced Cap-Score value resulted from an alteration in labeling. Samples not exposed to capacitation stimuli were compared and no difference was observed. Control and enzymatically liquefied samples had Cap-Score values of 22±4 and 21±5 (n=5; NC). These data support the view that treating semen with chymo, although widely used in clinical practice, can inhibit the ability of sperm to respond to capacitation stimuli.

Classes of compounds which may similarly interfere with the ability of sperm to respond to capacitation stimuli include: (i) a protease, including but not limited to, chymotrypsin, trypsin, collagenase, bromelain; (ii) a nucleases, including but not limited to, Dornase, HindIII, EcoRI; (iii) a mucolytic agent, including but not limited to, Erdostein, Acetylcysteine, Guiafenesin; (iv) a lipase, including but not limited to, Phospholipase Al, Phospholipase C, Lipoprotein lipase; (v) an esterase, including but not limited to, Cholinesterase, Thioesterase, Alkaline phosphatase; and (vi) Glycoside hydrolases, including but not limited to, Alpha-amylase, beta-galactosidase, hyaluronidase, neuorminodases, and lysozyme.

Liquefaction times of up to 2 hours and mechanical liquefaction using WOTP and/or PP did not influence capacitation. In contrast, the use of enzymes such as chymo reduced the ability of sperm to capacitate, as measured by Cap-Score™ Test. These results demonstrate the importance of knowing how semen processing methods impact sperm function.

Precision was evaluated by comparing the percent change about Cap-Score values (%Δ=(y₂-y₁)/y₂) when 50, 100, 150 and 200 sperm were evaluated. Changes in values of 11, 6 and 5% were observed for each addition of 50 sperm (n≥23). To be conservative, Cap-Score value was determined by counting the G_{M1} localization patterns of at least 150 cells. To assess variation within and between readers, 8 large image files containing up to 5,000 sperm were generated by combining images taken from multiple visual fields. Two different readers were trained and they determined Cap-Score values by randomly resampling each image 20 times, counting 150 cells each time. When scoring the same sample, individual readers reported an average SD of three (3) Cap-Score units. The difference between readers when scoring the same sample ranged from 0.00 to 1.52, with an average difference of one (1) between the readers for any given sample. Applying the Bonferroni correction for multiple comparisons, no difference between readers was observed for any image file (p-values ranged from 0.02 to 0.99).

Cap-Score was not affected by liquefaction time or mechanical liquefaction with WOTP or PP (n=5), though after washing and incubation, samples that had undergone two (2) hours liquefaction or passage through a PP showed a greater decline in motility (p=0.02, 3E-3). Use of a needle damaged sperm and was not investigated further. Liquefaction with chymo reduced Cap-Scores in CAP (p=0.03), but not NC samples (p=0.74). Samples incubated with chymo (n=5; 3mg/ml) could not be scored due to membrane damage, yet showed an increase in motility under NC conditions (p=9E-4).

### Example 2

This example was conducted using a cohort comparison between fertile (cohort 1, pregnant or recent father) and potential subfertile/infertile men (cohort 2, men questioning fertility). Relationships between Cap-Score and traditional semen measures were also explored.

All studies approved by WIRB (20152233). Semen samples were liquefied, washed, and incubated under non-capacitating and capacitating conditions. Sperm were fixed overnight and Cap-Score determined via fluorescence microscopy. Semen quality measures were evaluated according to WHO. T-Test, ANOVA and correlation analyses were done using Microsoft Excel (2013) and XLSTAT.

The mean Cap-Score for cohort 1 was 35.3 (SD= 7.7%; n=76 donors; 187 collections). Cap-Scores were lower for cohort 2 (p=1.0E-03), with 33.6% (41/122) having Cap-Scores below one (1) SD below the mean for cohort 1, versus an expected 16%. For cohort 2, no relationship was observed between Cap-Score and morphology (p=0.28), motility (p=0.14) or concentration (p=0.67). 93.4% (114/122) of men in cohort 2 exhibited normal motility, yet 30.7% (35/114) of them had Cap-Scores below one (1) SD below the mean. Similarly, 101 of 122 men (82.7%) exhibited normal concentration with 32.6% (33/101) having Cap-Scores below one (1) SD below the mean. These results show that capacitation defects are common in men having difficulty conceiving and the Cap-Score provides functional data that complement semen analysis.

The ability of sperm to capacitate differs between fertile men and those having trouble conceiving. Because capacitation is required for fertilization, the Cap-Score can provide an important functional complement to standard semen analysis and may help in choosing the most appropriate fertility treatment.

Common measures of semen quality are subjective and can vary within and among readers, making the assessment of male fertility challenging. The Cap-Score™ Test evaluates the ability of sperm to capacitate, a necessity for male fertility. The data presented here show that the Cap-Score™ Test is highly reproducible and reliable within and between readers, which are key considerations when attempting to diagnose male infertility.

The Cap-Score mean (µ=39) and SD (σ=7) from 41 fertile men were used to estimate the number of known fertile men needed for establishing a robust fertile capacitation profile. For a power analysis, an acceptable range about the mean was set at 3% and a two-tailed t-test at the p<0.01 level, with a probability of detecting a difference this large of 90% were applied. Results suggested that a valid standard can be established with ≥85 individuals. A preliminary normal fertile standard was created using 125 observations from 41 unique donors. The Cap-Score values were averaged by donor and then converted to z-scores ((X-µ)/σ; X=observation, µ=39; σ=7). This transformed the µ to 0 and the σ to 1, with converted values representing the distance from the µ (mean) in units of σ (S.D.). The normal fertile standard was tested against Cap-Score values from 93 men seeking fertility exams. This cohort scored significantly below the fertile population (p=1.6E-5), with 27 and 38% having z-scores ≤-2 and between -1 and -2. Only 35% scored near or above the mean. These data suggest that, in comparison to fertile men, many men seeking fertility exams have defects in capacitation.

### Example 3

The procedures used in Examples 1 and 2 were used in Example 3.

Classic semen analyses provide little information on the ability of samples to fertilize and egg. Capacitation is required for fertilization and can be assessed using G_{M1} localization. A comparison of the Cap-Score values from two cohorts of men revealed significant differences in their ability to capacitate. A robust capacitation profile can be defined and employed for identifying abnormalities. Remarkably, 33% of men questioning their fertility had z-scores ≤-1, versus an expected result of 16%. Combining the Cap-Score™ Test with traditional analyses should prove valuable in diagnosing male infertility.

Samples from 122 men referred to an infertility specialist were analyzed and had Cap-Scores ranging from 13 to 52%. An analysis of variance was done to compare Cap-Score values and sperm morphology. Samples were classified as having 0, 1, 2, 3, or ≥ 4% normal forms (scores ≥ 4% are considered normal, WHO) and mean Cap-Scores were compared among the groups. No relationship between Cap-Score value and morphology was observed (P=0.67). Next, sperm concentration (range 3x10⁶ to 210x10⁶/mL) was compared to Cap-Score value using the Pearson product-moment correlation coefficient and no connection was found (r=0.01, P=0.90). Lastly, Cap-Score value was compared to total % motility (range 15 to 80%) and the two measures were determined to be independent (r=0.14 P=0.21). Multiple donors who were classified as normal by WHO criteria had Cap-Score values more than two (2) SD below the normal mean, supporting the view that even normal appearing sperm can have functional abnormalities.

Traditional semen analysis identifies only 50% of male infertility cases. This study shows that there is little relationship between Cap-Score value and standard semen analysis parameters. Since capacitation is necessary for fertilization, the addition of the Cap-Score test to traditional semen evaluations could both identify cases of idiopathic infertility and help clinicians counsel couples towards the most appropriate treatment

The data presented herein demonstrate that a male's Cap-Score value may provide guidance on an appropriate mechanism for achieving successful mammalian pregnancies, including recommended assisted reproductive technology such as *in vitro* fertilization (IVF), or intracytoplasmic sperm injection (ICSI). The male may be a human or a non-human mammal. The Cap-Score value in combination with other components of a semen analysis, including concentration, total motility, progressive motility, volume, pH, viscosity and/or morphology may be considered. For example, the recommended assisted reproductive technology for two males with the same Cap-Score may differ if their sperm counts or sperm motility differ. In addition, the fertility status or reproductive health of the female partner would also be considered by the clinician.

### Example 4

Semen samples from consenting men were liquefied, washed and aliquots incubated under non-capacitating (NC) or capacitating (CAP) conditions. The consenting men included men who were classified as fertile based on a pregnant partner or the male being a recent biological father. The consenting men also included men seeking fertility exams. Capacitation conditions include *in vitro* exposure to 2-hydroxypropyl-β-cyclodextrin. Non-capacitation conditions include lack of *in vitro* exposure to any of bicarbonate ions, calcium ions and a mediator of sterol efflux such as 2-hydroxypropyl-β-cyclodextrin for varying periods of time. The *in vitro* capacitated sperm and the *in vitro* non-capacitated sperm were then fixed in a fixative such as paraformaldehyde or glutaraldehyde. The fixed *in vitro* capacitated sperm and the *in vitro* non-capacitated sperm were then labeled with a fluorescent labeled cholera toxin b subunit.

For one dataset, the sperm samples were incubated in capacitation or non-capacitation conditions for three (3) hours, fixed, labeled and then analyzed ("day0"). For a second dataset, the sperm samples were incubated in capacitation or non-capacitation conditions for three (3) hours, fixed overnight, labeled and then analyzed ("day1"). For a third dataset, the sperm samples were incubated in capacitation or non-capacitation conditions for three (3) hours, fixed, labeled and then analyzed. For a third dataset, the sperm samples were incubated in capacitation or non-capacitation conditions for 24 hours, fixed, labeled and then analyzed ("24hrCap"). Sperm capacitation was assessed using localization of G_{M1} (Cap-Score™).

102 sperm samples from 36 fertile men were evaluated at day0 and day1. Between day0 and day1, an increase in Cap-Score was observed in 81% (83/102) of sperm samples, with 44% of the sperm samples (45/102) having an increase in in Cap-Score of more than one (1) standard deviation (7%).

Sperm samples from 17 men seeking fertility treatment were evaluated at day0 and day1. Between day0 and day1, an increase in Cap-Score was observed in 29% (5/17) where the Cap-Scores increased more than one (1) standard deviation (7%).

To determine whether this change in Cap-Score was physiological or an artifact of being in fixative overnight, semen samples from nine (9) fertile men were analyzed at day0, day1 and after 24 hours of *in vitro* incubation in capacitation or non-capacitation medium and then fixed. All *in vitro* non-capacitated samples were equivalent (Cap-Scores of 19±2, 23±2 and 20±1%) and were different from the *in vitro* capacitated samples (Cap-Scores of 28±1, 34±2 and 31±2%, respectively). The Cap-Scores on day1 were significantly greater than the Cap-Scores for day0 (p=0.03). However, the Cap-Scores for *in vitro* capacitated samples incubated overnight in the fixative (day1) or in capacitating medium (24hrCap) were the same (p=0.33).

Consistent with prior literature, these data show that sperm membrane changes involved in capacitation still occur over time in certain fixatives. These data suggest that sperm achieve capacitation at different times in different ejaculates. To see if this was reproducible for an individual, 91 samples from 25 fertile men were classified as either early or late capacitators (day1-day0>7). The average concordance of change within donors was 76%, showing that capacitation timing was highly consistent within men.

### Example 5

Semen samples from 8 fertile men (pregnant partner or recent father) were used to examine the effect of cryopreservation on capacitation timing. Liquefied ejaculates were split; half processed immediately (fresh) and the other cryopreserved in test yolk buffer with glycerol (Irvine Scientific). Cryopreserved samples were subsequently thawed and processed (CryoT). Fresh and CryoT aliquots were washed and then incubated under non-capacitating (NC) and capacitating (CAP) conditions for 3 hours. Capacitation timing differs among men and can be evaluated by comparing Cap-Score differences from day 1 (after overnight incubation under conditions that promote/allow capacitation) to day 0 (analyzed after 3 hours incubation).

Cap-Score increased in NC CryoT treatments for both day 0 and day 1 when compared to fresh. For day 0 there was a 155% increase ((fresh - CryoT)/fresh; 11±1.6% vs 28±2.4%; n=7; p=0.00) and a 79% increase for day 1 (19±2.6% vs 34±2.7%; n=8; p=0.00). Conversely, Cap-Score for CAP treatments remained the same for both day 0 (26±3.1% vs 30±2.5%; n=7; p=0.31,) and day 1 (34±2.9% vs 34±1.7%; n=8; p=0.86,). Average post-thaw and post wash motilities of 27±3.5% and 31±8.6% for CryoT samples suggest reasonable post-thaw viability. When samples for day 0 and day 1 were compared, no difference in capacitation timing (day 1 - day 0; n=7) was observed between fresh and CryoT samples for NC (10±2.2% vs 8±2.7%) or CAP treatments (8±3.8% vs 5±2.3%).

### Example 6

All procedures, for specimen collection, were approved by WIRB (Protocol #20152233). Semen samples were collected from consenting men by manual masturbation after a minimum of 2 and a maximum of 5 days of sexual abstinence. Those samples having fewer than 10x10⁶ motile sperm cells were discarded from this study.

Ejaculates were liquefied at 37°C for at least 15 minutes and for no more than two (2) hours. Subsequent to liquefaction, the sperm were removed from the seminal plasma by centrifugation through Enhance S-Plus Cell Isolation Media (Vitrolife, reference: 15232 ESP-100-90%) at 300xg for 10 minutes. The cells were collected, resuspended in ∼4ml of Modified Human Tubal Fluid medium (mHTF; Irvine Scientific; reference 90126) and pelleted at 600xg for 10 minutes. The sperm were resuspended in mHTF with and without capacitation stimuli and incubated for three (3) hours. Following incubation, the samples were fixed as described (Selvaraj V, et al., "Segregation of micron-scale membrane sub-domains in live murine sperm," J Cell Physiol. 206: 636-46 (2006)) with paraformaldehyde (Electron Microscopy Sciences; Hatfield, PA) for at least 30 minutes prior to labeling.

Samples were labeled with 2 µg/mL of Cholera Toxin B, conjugated with Alexa Fluor 488 (CTB; Thermo Fisher: C34775). After ten minutes, 5 µl of the labeled sperm were placed on a microscope slide, overlaid with a cover slip and moved to an imaging station.

Imaging stations consisted of Nikon Eclipse NI-E microscopes equipped with: CFI60 Plan Apochromat Lambda 40x Objectives, C-FL AT GFP/FITC Long Pass Filter Sets, Hamamatsu ORCA-Flash 4.0 cameras, H101F - ProScan III Open Frame Upright Motorized H101F Flat Top Microscope Stages, and 64bit imaging workstations running NIS Elements software (Nikon; Melville, NY). These systems were programmed to automatically capture sets of 15x15 stitched images containing up to 5,000 sperm.

The Cap-Score SFT detects and analyzes localization patterns of the ganglioside G_{M1}. Two independent readers were trained to identify G_{M1} localization patterns that have been associated with capacitation of human sperm. The proportion of sperm within a sample having undergone capacitation was determined and reported as the Cap-Score.

Power Analysis was done using G*power (Faul et al., "G* Power 3: A flexible statistical power analysis program for the social, behavioral, and biomedical sciences," Behav Res Methods. 39: 175-91 (2007)). Student's t-Test was done using Microsoft Excel (2013). Linear Regression and Bartlett's test of homoscedasticity were carried out in XLSTAT Version 2015.5.01.22912.

### RESULTS

The first step in determining Cap-Score precision was to define the number of cells to count per sample. In general, as the number of cells counted increases, there is an increase in precision. However, at some point counting additional cells becomes redundant, as the Cap-Score will not change with additional observations. To identify the point when counting additional cells is unnecessary, the percent change about Cap-Score when 50, 100, 150 and 200 sperm was evaluated (Table 1).

**Table 1: Percent change in Cap-Score with increasing number of counted sperm.**

| **No. of counted sperm** | **Mean %Δ** | **STDEV** | **No. of Obs.** | **SEM** | **95% CI** | |
|---|---|---|---|---|---|---|
| | | | | | **LL** | **UL** |
| from 50 to 100 | 11% | 9% | 23 | 2% | 7% | 14% |
| from 100 to 150 | 6% | 5% | 26 | 1% | 4% | 8% |
| from 150 to 200 | 5% | 3% | 26 | 1% | 4% | 6% |

The percent change in Cap-Score when counting 50 or 100 sperm was large when compared to the percent change when counting 100 or 150 and 150 or 200 sperm. These observations supported the view that Cap-Score precision was only modestly improved by counting more than 100 sperm. However, the 95% confidence intervals for the percent change when counting 50 or 100 and 150 or 200 did not overlap, suggesting a significant reduction in percent change when at least 150 cells were counted. To be conservative, Cap-Score was determined by counting the G_{M1} localization patterns of at least 150 cells.

To further explore Cap-Score reliability and assess its potential as an objective measure of semen quality, its measurement was investigated to determine its accuracy within individual readers. Accuracy is defined as the proximity of measurements to the true value. The true value of an unknown population can be estimated by its central tendency, or the mean. One can judge whether a data set has a strong or a weak central tendency based on its dispersion, or the inverse of precision (JCGM/WG2 2008). That is to say that as dispersion increases, there is a decrease in precision. The standard deviation (SD) and Coefficient of Variation (CoV) measure the amount of dispersion within a sample. A standard deviation close to zero (0) indicates that the data points tend to be clustered tightly about the mean, while a high standard deviation indicates that the data points are spread out. Similarly, the CoV represents the amount of dispersion relative to the mean (CoV=SD/mean) and is useful for comparing the degree of variation from one data series to another, even if the means are drastically different from each other.

Prior to evaluating Cap-Score accuracy within readers, the number of images for each reader to sample was estimated. To this end, two semen donor groups were defined based on a cut-off of one (1) SD below the mean Cap-Score for a population of men with presumed fertility (pregnant wife or child less than 3 years old). The mean Cap-Score for the group with "lower Cap-Scores" was 27 and the "presumed fertile" group was 40. The standard deviations for each group were 5.2 and 4.9 respectively. A power analysis using a two-tailed test was done at the p<0.05 and p<0.01 level, with a probability of detecting a difference this large, if it exists, of 90% (1-beta=0.90). The results of these analyses indicated that sample 10 and 14 images respectively (5 and 7 per group) should be sampled. To be conservative, 10 images, each in the "lower Cap-Score" and "presumed fertile" groups, were generated. Sampling this number of images per group ensured that each was sufficiently interrogated to identify any differences in reproducibility that might occur because of either low- or high-value Cap-Scores.

To evaluate the accuracy of the Cap-Score SFT, two different readers determined Cap-Score by randomly resampling 10 images, that contained up to 5,000 sperm each, from the "lower Cap-Score" and "presumed fertile" groups and resampled 20 times by each reader. The SD and CoV were calculated on a per image basis for each reader. The average SD across images and readers was 3 (Fig 1A) and the average CoV was 13% (Fig 1B). Both the SD and CoV showed a linear relationship to Cap-Score. Thus, while there was greater dispersion associated with reading higher Cap-Scores, this appeared to result from a greater Cap-Score magnitude. These data were consistent with a high degree of accuracy, because when the same population of sperm was randomly resampled by the same or different reader, Cap-Score values were clustered tightly about the true value. Since these measure of variance and (or) dispersion are small and stable, they reveal a high degree of Cap-Score reproducibility within readers.

In general, a distribution can be described using its mean and variance. The mean indicates the location of the distribution, while the variance describes how dispersed the data are. One can envisage two distributions where the means are the same, yet the variances are different. For example, one distribution might resemble a normal bell shape, while the other is flatter having more extreme values. To demonstrate similar Cap-Score distributions between readers, 10 stitched images were obtained each for the "lower Cap-Score" and "presumed fertile" groups. Two different readers determined Cap-Scores by randomly resampling each image 20 times. Since each image file contained several magnitudes more sperm than were being sampled, each random resampling represented a distinct subsample of cells from within an individual ejaculate.

An average difference of one (1) in mean Cap-Score was observed between the readers for the 20 different images (Fig. 3). When the Bonferroni correction was applied, no discernable differences were observed. Similarly, Cap-Score variances were not different between readers (Fig. 3). These data support the view that Cap-Score was reproducible between readers, as independent readers obtained similar Cap-Score distributions when resampling the same population of sperm. Collectively, these data provide strong evidence that the Cap-Score SFT is highly reproducible and reliable within and between readers, which are key considerations when attempting to evaluate male reproductive fitness.

The data presented in the current study demonstrate that the Cap-Score SFT is highly reproducible and reliable within and between readers. The data and image files acquired should serve as a foundation for the continued quality control (QC) and quality assurance (QA) within and among laboratories in the evaluation of Cap-Score. For example, two of the 20 image files, one each from the "lower Cap-Scores" and "presumed fertile" groups could be selected at random and scored each day to demonstrate a reader's daily ability to read Cap-Scores. If values are obtained that are outside of acceptable ranges from the established mean (Westgard et al., "A multi-rule Shewhart chart for quality control in clinical chemistry," Clin Chem. 27: 493-50 (1981), the laboratory director can be consulted for remediation. These data can also be used to track individual readers over time and to identify potential changes in Cap-Score determination. Similarly, as new personal and (or) laboratories are trained and incorporated into the reading rotation, their reading ability can be evaluated by scoring multiple image files and comparing their Cap-Scores to established values. Such an approach would ensure comparable data both within and among laboratories. Only through continued internal and external QA and QC can high standards of sperm function evaluations be maintained.

Male fertility diagnosis has historically been plagued by the inability to assess sperm function; namely, the ability to fertilize (Oehninger et al., "Sperm functional tests," Fertil Steril. 102: 1528-33 (2014); Wang et al., "Limitations of semen analysis as a test of male fertility and anticipated needs from newer tests," Fertil Steril. 102: 1502-07 (2014)). Such a diagnostic capability would provide a functional complement to the descriptive assessments of traditional semen evaluations. Identifying sperm with deficiencies in fertilizing ability may allow for a more specific diagnosis of what is now categorized as idiopathic infertility. Of much greater practical importance, this information could enable a clinician to help counsel a couple toward the most appropriate form of ART to achieve pregnancy. To achieve the previously specified goal, many assays of sperm function have been suggested (e.g., hamster zona penetration assays (Barros et al., "Human Sperm Penetration into Zona-Free Hamster Oocytes as a Test to Evaluate the Sperm Fertilizing Ability," Andrologia. 11: 197-210 (1979); Rogers et al., "Analysis of human spermatozoal fertilizing ability using zona-free ova," Fertil Steril. 32: 664-70)1979), sperm-ZP binding tests (Liu et al., "Clinical application of sperm-oocyte interaction tests in in vitro fertilization-embryo transfer and intracytoplasmic sperm injection programs," Fertil Steril. 82: 1251-63 (2004), and cervical mucus penetration assays (Alexander, "Evaluation of male infertility with an in vitro cervical mucus penetration test," Fertil Steril. 36: 201-8 (1981); Menge et al., "Interrelationships among semen characteristics, antisperm antibodies, and cervical mucus penetration assays in infertile human couples." Fertil Steril. 51: 486-92 (1989); Eggert-Kruse et al., "Prognostic value of in vitro sperm penetration into hormonally standardized human cervical mucus," Fertil Steril. 51: 317-23 (1989). However, their use has been limited by the great difficulty in obtaining needed materials in a logistically practical fashion. To fill the current void, a diagnostic tool has been developed to evaluate the ability of sperm to undergo the physiological changes required to fertilize an oocyte.

## Claims

1. A method for identifying an approach for achieving mammalian fertilization comprising the steps of:
obtaining by imaging one or more t₀-fluorescence images from a first sample of *in vitro* capacitated sperm cells treated with a fluorescence label, wherein the sperm cells are displaying one or more G_{M1} localization patterns,
wherein said t₀-fluorescence images are obtained at post *in vitro* capacitation times selected from: 0.1 hour to 5 hours; 0.1 hour to 8 hours; 0.1 to 12 hours; or 0.1 hour to 18 hours;
measuring a number of apical acrosome (AA) G_{M1} localization patterns, a number of acrosomal plasma membrane (APM) G_{M1} localization patterns and a total number of G_{M1} localization patterns displayed in one or more to-fluorescence images to determine a percentage of to-[AA G_{M1} localization patterns plus APM G_{M1} localization patterns],
comparing the percentage of measured to-[AA G_{M1} localization patterns plus APM G_{M1} localization patterns] to a reference percentage of [AA G_{M1} localization patterns plus APM G_{M1} localization patterns] to determine a fertility status; and
identifying a reproductive approach for achieving mammalian fertilization based on the determined fertility status,
wherein a reference percentage of [AA G_{M1} localization patterns plus APM G_{M1} localization patterns] corresponding to: greater than 35 % indicates a high fertility status; one standard deviation below 35 % indicates a medium fertility status; and two or more standard deviations below 35 % indicates a low fertility status.

2. The method of claim 1, wherein if the male has at least normal sperm concentration, then
the reproductive approach for high fertility status is selected from the group consisting of: intercourse, intracervical insemination (ICI), pre-capacitating sperm prior to intracervical insemination, intrauterine insemination (IUI), or pre-capacitating sperm prior to intrauterine insemination;
the reproductive approach for medium fertility status is selected from the group consisting of: intracervical insemination (ICI), pre-capacitating sperm prior to intracervical insemination; intrauterine insemination (IUI); pre-capacitating sperm prior to intrauterine insemination; or *in vitro* fertilization (IVF) or pre-capacitating sperm prior to *in vitro* fertilization; or
the reproductive approach for low fertility status is selected from group consisting of: *in vitro* fertilization (IVF), pre-capacitating sperm prior to *in vitro* fertilization, intracytoplasmic sperm injection (ICSI), pre-capacitating sperm prior to intracytoplasmic sperm injection, gamete intra-fallopian transfer (GIFT), pre-capacitating sperm prior to gamete intra-fallopian transfer, subzonal insemination (SUZI), or pre-capacitating sperm prior to subzonal insemination.

3. The method according to claim 2, wherein if the reproductive approach corresponds to pre-capacitating sperm prior to *in vitro* fertilization, intracytoplasmic sperm injection (ICSI), gamete intra-fallopian transfer (GIFT), or subzonal insemination (SUZI), then the time period for pre-capacitation prior to insemination corresponds to incubating sperm in media containing one or more stimuli for capacitation, for periods of 24 hours before insemination; 18 hours before insemination; 12 hours before insemination; 6 hours before insemination; 4 hours before insemination; 3 hours before insemination; or 1 hour before insemination.

4. The method according to claim 3, wherein the method further comprises the steps of:
obtaining by imaging one or more ti-fluorescence images from a second sample of *in vitro* capacitated sperm cells obtained from the same male as the first sample and treated with a fluorescence label displaying one or more G_{M1} localization patterns, wherein said ti-fluorescence images are obtained at post capacitation time ti, wherein ti is greater than to or greater than 18 hours;
measuring a number of apical acrosome (AA) G_{M1} localization patterns, a number of acrosomal plasma membrane (APM) G_{M1} localization patterns and a total number of G_{M1} localization patterns displayed in one or more ti-fluorescence images to determine a percentage of t₁-[AA G_{M1} localization patterns plus APM G_{M1} localization patterns];
comparing the percentage of t₁-[AA G_{M1} localization patterns plus APM G_{M1} localization patterns] to the percentage of t₀-[AA G_{M1} localization patterns plus APM G_{M1} localization patterns] to determine an *in vivo* capacitation time,
wherein a difference in the percentage of t₀-[AA G_{M1} localization patterns plus APM G_{M1} localization patterns] and the percentage of t₁-[AA G_{M1} localization patterns plus APM G_{M1} localization patterns] of greater than one standard deviation from a standard of 35 % indicates an *in vivo* capacitation time greater than 12 hours; and a difference of less than one standard deviation from the standard of 35 % indicates an *in vivo* capacitation time of less than 12 hours;
further if the *in vivo* capacitation time is determined to be greater than 12 hours, then a t₁-fertility status is determined based on a comparison of the percentage of measured t₁-[AA G_{M1} localization patterns plus APM G_{M1} localization patterns] to the reference percentage of [AA G_{M1} localization patterns plus APM G_{M1} localization patterns]; or, if the *in vivo* capacitation time is determined to be less than 12 hours, then a ti-fertility status is determined based on a comparison of the reference percentage of [AA G_{M1} localization patterns plus APM G_{M1} localization patterns] to the percentage of measured t₁-[AA G_{M1} localization patterns plus APM G_{M1} localization patterns] or the percentage of measured t₀-[AA G_{M1} localization patterns plus APM G_{M1} localization patterns]; and
based on the male's ti-fertility status and *in vivo* capacitation time, identifying a time period for insemination and a reproductive approach to use in order to achieve fertilization.

5. The method according to any preceding claim, wherein the identifying step is also based on one or more of the following: patient demographics, reproductive status of female partner, sperm concentration, total motility, progressive motility, semen volume, semen pH, semen viscosity and/or sperm morphology and combinations thereof.

6. The method according to any preceding claim, wherein the more than one G_{M1} localization patterns include AA G_{M1} localization pattern, APM G_{M1} localization pattern, Lined-Cell G_{M1} localization pattern, INTER G_{M1} localization pattern, PAPM G_{M1} localization pattern, AA/PA G_{M1} localization pattern, ES G_{M1} localization pattern, and DIFF G_{M1} localization pattern.

7. The method of any preceding claim, wherein the sperm cells have been treated *in vitro* with capacitation conditions for a capacitation time period of: at least one hour; at least 3 hours; at least 12 hours; at least 18 hours; at least 24 hours; for a capacitation time period ranging between 0.5 hours to 3 hours; 3 hours to 12 hours; 6 hours to 12 hours; 3 hours to 24 hours; 12 hours to 24 hours; or 18 hours to 24 hours.

8. The method of any preceding claim, wherein the *in vitro* capacitated sperm cells have been treated with a fixative for a fixative time period of: at least 0.5 hour; at least 3 hours; at least 12 hours; at least 18 hours; at least 24 hours; at least 30 hours; at least 36 hours; or at least 48 hours, for a fixation time period ranging between 0.5 hours to 3 hours; 3 hours to 12 hours; 6 hours to 12 hours; 3 hours to 18 hours; 6-18 hours; 6-24 hours; 12 hours to 24 hours; 18 hours to 24 hours; 18-30 hours; 18-36 hours; 24-30 hours; 24-26 hours; 18-48 hours; 24-48 hours; or 36-48 hours.

9. The method of any preceding claim, wherein the sperm cells are treated to cryopreservation procedures and stored prior to being treated *in vitro* with capacitation conditions.

10. The method of claim 1, wherein if the male has a less than normal sperm concentration, then
the reproductive approach for high fertility status is selected from group consisting of: intracervical insemination (ICI), pre-capacitating sperm prior to intracervical insemination, intrauterine insemination (IUI) or pre-capacitating sperm prior to intrauterine insemination;
the reproductive approach for medium fertility status is selected from group consisting of: intracervical insemination (ICI), pre-capacitating sperm prior to intracervical insemination, intrauterine insemination (IUI) or pre-capacitating sperm prior to intrauterine insemination; in vitro fertilization (IVF), pre-capacitating sperm prior to in vitro fertilization; or
the reproductive approach for low fertility status is selected from group consisting of: in vitro fertilization (IVF), pre-capacitating sperm prior to in vitro fertilization, intracytoplasmic sperm injection (ICSI), pre-capacitating sperm prior to intracytoplasmic sperm injection, gamete intra-fallopian transfer (GIFT), pre-capacitating sperm prior to gamete intrafallopian transfer, subzonal insemination (SUZI) or pre-capacitating sperm prior to subzonal insemination.

## Patentansprüche

1. Verfahren zur Identifizierung eines Ansatzes zur Erzielung von Säugetierbefruchtung, das die folgenden Schritte umfasst:
Erhalten einer oder mehrerer t₀-Fluoreszenzaufnahmen mittels Bildgebungsverfahren von einer ersten Probe von *in vitro* kapazitierten Spermazellen, die mit einem Fluoreszenzmarker behandelt wurden, wobei die Spermazellen eine oder mehr G_{M1}-Lokalisierungsmuster zeigen,
wobei die t₀-Fluoreszenzaufnahmen zu *post-In-vitro-*Kapazitationszeiten erhalten werden, die ausgewählt sind aus: 0,1 Stunde bis 5 Stunden; 0,1 Stunde bis 8 Stunden; 0,1 Stunde bis 12 Stunden; oder 0,1 Stunde bis 18 Stunden;
Messen von mehreren apikalen Akrosom (AA) G_{M1}-Lokalisierungsmustern, mehreren akrosomalen Plasmamembran (APM) G_{M1}-Lokalisierungsmustern und einer Gesamtzahl von G_{M1}-Lokalisierungsmustern, die in einer oder mehreren t₀-Fluoreszenzaufnahmen gezeigt werden, um einen Prozentsatz von t₀- [AA-G_{M1}-Lokalisierungsmustern plus APM-G_{M1}-Lokalisierungsmustern] zu ermitteln,
Vergleichen des Prozentsatzes von gemessenen t₀-[AA-G_{M1}-Lokalisierungsmustern plus APM-G_{M1}-Lokalisierungsmustern] mit einem Referenzprozentsatz von [AA-G_{M1}-Lokalisierungsmustern plus APM-G_{M1}-Lokalisierungsmustern], um einen Fertilitätsstatus zu ermitteln; und
Identifizieren eines Fortpflanzungsansatzes zur Erzielung von Säugetierbefruchtung basierend auf dem ermittelten Fertilitätsstatus,
wobei ein Referenzprozentsatz von [AA-G_{M1}-Lokalisierungsmustern plus APM-G_{M1}-Lokalisierungsmustern] entsprechend: mehr als 35 % einen hohen Fertilitätsstatus anzeigt; eine Standardabweichung von weniger als 35 % einen mittleren Fertilitätsstatus anzeigt; und zwei oder mehr Standardabweichungen von weniger als 35 % einen niedrigen Fertilitätsstatus anzeigt.

2. Verfahren nach Anspruch 1, wobei - wenn der Mann bzw. das Männchen mindestens eine normale Spermazellkonzentration aufweist -
der Fortpflanzungsansatz bei hohem Fertilitätsstatus ausgewählt ist aus der Gruppe bestehend aus: Geschlechtsverkehr, intrazervikaler Insemination (intracervical insemination, ICI), Präkapazitation von Spermazellen vor einer intrazervikalen Insemination, intrauteriner Insemination (intrauterine insemination, IUI) oder Präkapazitation von Spermazellen vor einer intrauterinen Insemination;
der Fortpflanzungsansatz bei mittlerem Fertilitätsstatus ausgewählt ist aus der Gruppe bestehend aus: intrazervikaler Insemination (ICI), Präkapazitation von Spermazellen vor einer intrazervikalen Insemination, intrauteriner Insemination (IUI) oder Präkapazitation von Spermazellen vor einer intrauterinen Insemination; oder *In-vitro*-Fertilisation (IVF) oder Präkapazitation von Spermazellen vor einer In-vitro-Fertilisation, oder
der Fortpflanzungsansatz bei niedrigem Fertilitätsstatus ausgewählt ist aus der Gruppe bestehend aus: *In-vitro*-Fertilisation (IVF), Präkapazitation von Spermazellen vor einer In-vitro-Fertilisation, intrazytoplasmischer Spermazellinjektion (intracytoplasmic sperm injection, ICSI), Präkapazitation von Spermazellen vor einer intrazytoplasmischen Spermazellinjektion, intratubarem Gametentransfer (gamete intra-fallopian transfer, GIFT), Präkapazitation von Spermazellen vor einem intratubaren Gametentransfer, subzonaler Insemination (SUZI) oder Präkapazitation von Spermazellen vor einer subzonalen Insemination.

3. Verfahren nach Anspruch 2, wobei - wenn der Fortpflanzungsansatz einer Präkapazitation von Spermazellen vor einer In-vitro-Fertilisation, einer intrazytoplasmischen Spermazellinjektion (ICSI), einem intratubaren Gametentransfer (GIFT) oder einer subzonalen Insemination (SUZI) entspricht - die Zeitspanne für eine Präkapazitation vor einer Insemination einer Inkubation von Spermazellen in Medien entspricht, die einen oder mehrere Stimuli zur Kapazitation enthalten, über Zeiträume von 24 Stunden vor einer Insemination; 18 Stunden vor einer Insemination; 12 Stunden vor einer Insemination; 6 Stunden vor einer Insemination; 4 Stunden vor einer Insemination; 3 Stunden vor einer Insemination; oder 1 Stunde vor einer Insemination.

4. Verfahren nach Anspruch 3, wobei das Verfahren ferner die folgenden Schritte umfasst:
Erhalten von einer oder mehreren t₁-Fluoreszenzaufnahmen mittels Bildgebungsverfahren von einer zweiten Probe von *in vitro* kapazitierten Spermazellen, die von demselben Mann bzw. Männchen wie die erste Probe erhalten wurden und mit einem Fluoreszenzmarker, der ein oder mehrere G_{M1}-Lokalisierungsmuster zeigt, behandelt wurden, wobei die t₁-Fluoreszenzaufnahmen zur post-Kapazitationszeit t₁ erhalten werden, wobei t₁ größer als t₀ oder größer als 18 Stunden ist;
Messen von mehreren apikalen Akrosom (AA) G_{M1}-Lokalisierungsmustern, mehreren akrosomalen Plasmamembran (APM) G_{M1}-Lokalisierungsmustern und einer Gesamtzahl von G_{M1}-Lokalisierungsmustern, die in einer oder mehreren t₁-Fluoreszenzaufnahmen gezeigt werden, um einen Prozentsatz von t₁-[AA-G_{M1}-Lokalisierungsmustern plus APM-G_{M1}-Lokalisierungsmustern] zu ermitteln,
Vergleichen des Prozentsatzes von t₁-[AA-G_{M1}-Lokalisierungsmustern plus APM-G_{M1}-Lokalisierungsmustern] mit dem Prozentsatz von t₀-[AA-G_{M1}-Lokalisierungsmustern plus APM-G_{M1}-Lokalisierungsmustern] , um eine *In-vivo-*Kapazitationszeit zu ermitteln,
wobei eine Differenz zwischen dem Prozentsatz von t₀-[AA-G_{M1}-Lokalisierungsmustern plus APM-G_{M1}-Lokalisierungsmustern] und dem Prozentsatz von t₁-[AA-G_{M1}-Lokalisierungsmustern plus APM-G_{M1}-Lokalisierungsmustern] von mehr als einer Standardabweichung von einem Standard von 35% eine *In-vivo*-Kapazitationszeit von mehr als 12 Stunden anzeigt; und eine Differenz von weniger als einer Standardabweichung vom Standard von 35% eine *In-vivo-*Kapazitationszeit von weniger als 12 Stunden anzeigt;
wenn ferner die ermittelte *In*-*vivo*-Kapazitationszeit mehr als 12 Stunden beträgt, wird ein t₁-Fertilitätsstatus basierend auf einem Vergleich des Prozentsatzes von gemessenen t₁-[AA-G_{M1}-Lokalisierungsmustern plus APM-G_{M1}-Lokalisierungsmustern] mit dem Referenzprozentsatz von [AA-G_{M1}-Lokalisierungsmustern plus APM-G_{M1}-Lokalisierungsmustern] ermittelt; oder, wenn die ermittelte *In-vivo*-Kapazitationszeit weniger als 12 Stunden beträgt, wird ein t₁-Fertilitätsstatus basierend auf einem Vergleich des Referenzprozentsatzes von [AA-G_{M1}-Lokalisierungsmustern plus APM-G_{M1}-Lokalisierungsmustern] mit dem Prozentsatz von gemessenen t₁-[AA-G_{M1}-Lokalisierungsmustern plus APM-G_{M1}-Lokalisierungsmustern] oder dem Prozentsatz von gemessenen t₀-[AA-G_{M1}-Lokalisierungsmustern plus APM-G_{M1}-Lokalisierungsmustern] ermittelt, und
basierend auf dem t₁-Fertilitätsstatus des Mannes bzw. des Männchens und der *In-vivo*-Kapazitationszeit Identifizieren eines Zeitraumes für eine Insemination und eines Befruchtungsansatzes, der angewendet wird, um eine Fertilisation zu erzielen.

5. Verfahren nach einem vorhergehenden Anspruch, wobei der Identifizierungsschritt auch auf einem oder mehreren der Folgenden basiert: Patientendemographie, Fertilitätsstatus der weiblichen Partnerin, Spermazellkonzentration, Gesamtmotilität, progressive Motilität, Samenvolumen, Samen-pH, Samenviskosität und/oder Spermazellmorphologie und Kombinationen davon.

6. Verfahren nach einem vorhergehenden Anspruch, wobei die mehr als einen G_{M1}-Lokalisierungsmuster AA-G_{M1}-Lokalisierungsmuster, APM-G_{M1}-Lokalisierungsmuster, Lined-Cell-G_{M1}-Lokalisierungsmuster, INTER-G_{M1}-Lokalisierungsmuster, PAPM-G_{M1}-Lokalisierungsmuster, AA/PA-G_{M1}-Lokalisierungsmuster, ES-G_{M1}-Lokalisierungsmuster und DIFF-G_{M1}-Lokalisierungsmuster einschließen.

7. Verfahren nach einem vorhergehenden Anspruch, wobei die Spermazellen *in vitro* unter Kapazitationsbedingungen behandelt worden sind und zwar über einen Kapazitationszeitraum von: mindestens einer Stunde; mindestens 3 Stunden; mindestens 12 Stunden; mindestens 18 Stunden; mindestens 24 Stunden; über einen Kapazitationszeitraum im Bereich von 0,5 Stunden bis 3 Stunden; 3 Stunden bis 12 Stunden; 6 Stunden bis 12 Stunden; 3 Stunden bis 24 Stunden, 12 Stunden bis 24 Stunden; oder 18 Stunden bis 24 Stunden.

8. Verfahren nach einem vorhergehenden Anspruch, wobei die *in vitro* kapazitierten Spermazellen mit einem Fixiermittel behandelt worden sind und zwar über einen Fixierzeitraum von: mindestens 0,5 Stunden; mindestens 3 Stunden; mindestens 12 Stunden; mindestens 18 Stunden; mindestens 24 Stunden; mindestens 30 Stunden; mindestens 36 Stunden; oder mindestens 48 Stunden; über einen Fixierzeitraum im Bereich von 0,5 Stunden bis 3 Stunden; 3 Stunden bis 12 Stunden; 6 Stunden bis 12 Stunden; 3 Stunden bis 18 Stunden; 6-18 Stunden; 6-24 Stunden; 12 Stunden bis 24 Stunden; 18 Stunden bis 24 Stunden; 18-30 Stunden; 18-36 Stunden; 24-30 Stunden; 24-26 Stunden; 18-48 Stunden; 24-48 Stunden; oder 36-48 Stunden.

9. Verfahren nach einem vorhergehenden Anspruch, wobei die Spermazellen mit Kryokonservierungsverfahren behandelt werden und vor der In-vitro-Behandlung unter Kapazitationsbedingungen aufbewahrt werden.

10. Verfahren nach Anspruch 1, wobei - wenn der Mann bzw. das Männchen eine weniger als normale Spermazellkonzentration aufweist -
der Fortpflanzungsansatz bei hohem Fertilitätsstatus ausgewählt ist aus der Gruppe bestehend aus: intrazervikaler Insemination (ICI), Präkapazitation von Spermazellen vor einer intrazervikalen Insemination, intrauteriner Insemination (IUI) oder Präkapazitation von Spermazellen vor einer intrauterinen Insemination,
der Fortpflanzungsansatz bei mittlerem Fertilitätsstatus ausgewählt ist aus der Gruppe bestehend aus: intrazervikaler Insemination (ICI), Präkapazitation von Spermazellen vor einer intrazervikalen Insemination, intrauteriner Insemination (IUI) oder Präkapazitation von Spermazellen vor einer intrauterinen Insemination, *In-vitro-*Fertilisation (IVF), Präkapazitation von Spermazellen vor einer In-vitro-Fertilisation; oder
der Fortpflanzungsansatz bei niedrigem Fertilitätsstatus ausgewählt ist aus der Gruppe bestehend aus: In-vitro-Fertilisation (IVF), Präkapazitation von Spermazellen vor einer In-vitro-Fertilisation, intrazytoplasmischer Spermazellinjektion (ICSI), Präkapazitation von Spermazellen vor einer intrazytoplasmischen Spermazellinjektion, intratubarem Gametentransfer (GIFT), Präkapazitätion von Spermazellen vor einem intratubaren Gamententransfer, subzonaler Insemination (SUZI) oder Präkapazitation von Spermazellen vor einer subzonalen Insemination.

## Revendications

1. Procédé d'identification d'une approche permettant d'accomplir une fécondation chez un mammifère, comprenant les étapes suivantes :
obtention par imagerie d'une ou de plusieurs images de la fluorescence à t₀ à partir d'un premier échantillon de cellules spermatiques capacitées *in vitro* traitées avec un marqueur fluorescent, où les cellules spermatiques présentent un ou plusieurs profils de localisation du G_{M₁},
où lesdites images de la fluorescence à t₀ sont obtenues après des temps de capacitation *in vitro* sélectionnés parmi les suivants: 0,1 heure à 5 heures ; 0,1 heure à 8 heures ; 0,1 à 12 heures ; ou 0,1 heure à 18 heures ;
mesure d'un nombre de profils de localisation du G_{M₁} dans la zone apicale de l'acrosome (AA), d'un nombre de profils de localisation du G_{M₁} dans la membrane plasmique de l'acrosome (APM) et d'un nombre total de profils de localisation du G_{M₁} visualisés sur une ou plusieurs images de la fluorescence à t0 pour déterminer un pourcentage des [profils de localisation du GM₁ dans l'AA plus profils de localisation du G_{M1} dans l'APM] à to,
comparaison entre le pourcentage des [profils de localisation du G_{M₁} dans l'AA plus profils de localisation du G_{M₁} dans l'APM] mesuré à t₀ et un pourcentage de référence des [profils de localisation du G_{M1} dans l'AA plus profils de localisation du G_{M₁} dans l'APM] pour déterminer un état de fertilité ; et
identification d'une approche permettant d'accomplir une fécondation chez un mammifère sur la base de l'état de fertilité déterminé,
où un pourcentage de référence des [profils de localisation du G_{M₁} dans l'AA plus profils de localisation du G_{M₁} dans l'APM] correspondant à : plus de 35 % est indicateur d'un état de fertilité élevé ; un écart type en-dessous de 35 % est indicateur d'un état de fertilité moyen ; et deux ou plus écarts types en-dessous de 35 % est indicateur d'un état de fertilité faible.

2. Procédé de la revendication 1 où, si le mâle a une concentration spermatique au moins normale, l'approche reproductive utilisée si l'état de fertilité est élevé est sélectionnée dans le groupe consistant en les suivantes :
rapport sexuel, insémination intracervicale (ICI), pré-capacitation du sperme avant insémination intracervicale, insémination intra-utérine (IUI) ou pré-capacitation du sperme avant insémination intra-utérine ;
l'approche reproductive utilisée si l'état de fertilité est moyen est sélectionnée dans le groupe consistant en les suivantes : insémination intracervicale (ICI), pré-capacitation du sperme avant insémination intracervicale, insémination intra-utérine (IUI) ; pré-capacitation du sperme avant insémination intra-utérine ; ou fécondation *in vitro* (FIV) ou pré-capacitation du sperme avant fécondation *in vitro* ; ou
l'approche reproductive utilisée si l'état de fertilité est faible est sélectionnée dans le groupe consistant en les suivantes : fécondation *in vitro* (FIV), pré-capacitation du sperme avant fécondation *in vitro,* injection intracytoplasmique de spermatozoïdes (ICSI), pré-capacitation du sperme avant injection intracytoplasmique de spermatozoïdes, transfert intratubaire de gamètes (GIFT), pré-capacitation du sperme avant transfert intratubaire de gamètes, injection subzonale de spermatozoïdes (SUZI) ou pré-capacitation du sperme avant injection subzonale de spermatozoïdes.

3. Procédé selon la revendication 2 où, si l'approche reproductive correspond à une pré-capacitation du sperme avant fécondation *in vitro,* injection intracytoplasmique de spermatozoïdes (ICSI), transfert intratubaire de gamètes (GIFT) ou injection subzonale de spermatozoïdes (SUZI), la période de temps de pré-capacitation avant l'insémination correspond à l'incubation du sperme dans des milieux qui contiennent un ou plusieurs stimuli de la capacitation pendant des périodes d'une durée de 24 heures avant l'insémination ; de 18 heures avant l'insémination; de 12 heures avant l'insémination; de 6 heures avant l'insémination ; de 4 heures avant l'insémination ; de 3 heures avant l'insémination ; ou de 1 heure avant l'insémination.

4. Procédé selon la revendication 3, où le procédé comprend en outre les étapes suivantes :
obtention par imagerie d'une ou de plusieurs images de la fluorescence à t₁ à partir d'un deuxième échantillon de cellules spermatiques capacitées *in vitro* provenant du même mâle que le premier échantillon et traitées avec un marqueur fluorescent permettant de visualiser un ou plusieurs profils de localisation du G_{M₁}, où lesdites images de la fluorescence à t₁ sont obtenues après un temps de capacitation t₁, où t₁ est supérieur à t0 ou supérieur à 18 heures ;
mesure d'un nombre de profils de localisation du G_{M₁} dans la zone apicale de l'acrosome (AA), d'un nombre de profils de localisation du G_{M₁} dans la membrane plasmique de l'acrosome (APM) et d'un nombre total de profils de localisation du G_{M₁} visualisés sur une ou plusieurs images de la fluorescence à t₁ pour déterminer un pourcentage des [profils de localisation du G_{M₁} dans l'AA plus profils de localisation du G_{M₁} dans l'APM] à t₁ ;
comparaison du pourcentage des [profils de localisation du G_{M₁} dans l'AA plus profils de localisation du G_{M1} dans l'APM] à t₁ et du pourcentage des [profils de localisation du G_{M₁} dans l'AA plus profils de localisation du G_{M₁} dans l'APM] à t0 pour déterminer un temps de capacitation *in vivo,*
où une différence entre le pourcentage des [profils de localisation du G_{M₁} dans l'AA plus profils de localisation du G_{M₁} dans l'APM] à t0 et le pourcentage des [profils de localisation du G_{M₁} dans l'AA plus profils de localisation du G_{M₁} dans l'APM] à t₁ de plus de un écart type par rapport à une valeur standard de 35 % est indicatrice d'un temps de capacitation *in vivo* supérieur à 12 heures ; et une différence de moins de un écart type par rapport à la valeur standard de 35 % est indicatrice d'un temps de capacitation *in vivo* inférieur à 12 heures ;
de plus, s'il est établi que le temps de capacitation *in vivo* est supérieur à 12 heures, un état de fertilité à t₁ est déterminé sur la base d'une comparaison entre le pourcentage des [profils de localisation du G_{M₁} dans l'AA plus profils de localisation du G_{M₁} dans l'APM] mesuré à t₁ et le pourcentage de référence des [profils de localisation du G_{M1} dans l'AA plus profils de localisation du G_{M₁} dans l'APM] ; ou, s'il est établi que le temps de capacitation *in vivo* est inférieur à 12 heures, un état de fertilité à t₁ est déterminé sur la base d'une comparaison entre le pourcentage de référence des [profils de localisation du G_{M₁} dans l'AA plus profils de localisation du G_{M₁} dans l'APM] et le pourcentage des [profils de localisation du G_{M₁} dans l'AA plus profils de localisation du G_{M₁} dans l'APM] mesuré à t₁ ou le pourcentage des [profils de localisation du G_{M₁} dans l'AA plus profils de localisation du G_{M1} dans l'APM] mesuré à t₀ ; et
sur la base de l'état de fertilité du mâle à t₁ et d'un temps de capacitation *in vivo,* identification d'une période de temps pour l'insémination et d'une approche reproductive à utiliser pour parvenir à une fécondation

5. Procédé selon l'une quelconque des revendications précédentes, où l'étape d'identification repose également sur un ou plusieurs des suivants : données démographiques du patient, état reproducteur d'une partenaire de sexe féminin, concentration spermatique, motilité totale, motilité progressive, volume de semence, pH de la semence, viscosité de la semence et/ou morphologie des spermatozoïdes et combinations de ceux-ci.

6. Procédé selon l'une quelconque des revendications précédentes, où les plus d'un profil de localisation du G_{M₁} incluent un profil de localisation du G_{M₁} dans l'AA, un profil de localisation du G_{M₁} dans l'APM, un profil de localisation du G_{M₁} dans les cellules à membrane, un profil de localisation du G_{M₁} dans une zone dite intermédiaire (INTER), un profil de localisation du G_{M₁} dans la membrane plasmique post-acrosomique (PAPM), un profil de localisation du G_{M₁} dans la zone apicale de l'acrosome/la région post-acrosomique (AA/PA), un profil de localisation du G_{M₁} dans le segment équatorial (ES) et un profil de localisation diffuse du G_{M₁} (DIFF).

7. Procédé de l'une quelconque des revendications précédentes, où les cellules spermatiques ont été traitées *in vitro* dans des conditions de capacitation pendant une période de temps de capacitation sélectionnée parmi les suivantes : au moins une heure ; au moins 3 heures ; au moins 12 heures; au moins 18 heures; au moins 24 heures; pendant une période de temps de capacitation qui va de 0,5 heure à 3 heures ; de 3 heures à 12 heures ; de 6 heures à 12 heures ; de 3 heures à 24 heures ; de 12 heures à 24 heures ; ou de 18 heures à 24 heures.

8. Procédé de l'une quelconque des revendications précédentes, où les cellules spermatiques capacitées *in vitro* ont été traitées avec un fixateur pendant une période de temps de fixation sélectionnée parmi les suivantes : au moins 0,5 heure ; au moins 3 heures ; au moins 12 heures ; au moins 18 heures ; au moins 24 heures ; au moins 30 heures ; au moins 36 heures ; ou au moins 48 heures, pendant une période de temps de fixation qui va de 0,5 heure à 3 heures ; de 3 heures à 12 heures ; de 6 heures à 12 heures ; de 3 heures à 18 heures ; de 6-18 heures ; de 6-24 heures ; de 12 heures à 24 heures ; de 18 heures à 24 heures ; de 18-30 heures ; de 18-36 heures ; de 24-30 heures ; de 24-26 heures ; de 18-48 heures ; de 24-48 heures ; ou de 36-48 heures.

9. Procédé de l'une quelconque des revendications précédentes, où les cellules spermatiques ont été traitées par des procédures de cryopréservation et stockées avant d'être traitées *in vitro* dans des conditions de capacitation.

10. Procédé de la revendication 1 où, si le mâle a une concentration en spermatozoïdes inférieure à la normale,
l'approche reproductive utilisée si l'état de fertilité est élevé est sélectionnée dans le groupe consistant en les suivantes : insémination intracervicale (ICI), pré-capacitation du sperme avant insémination intracervicale, insémination intra-utérine (IUI) ou pré-capacitation du sperme avant insémination intra-utérine ;
l'approche reproductive utilisée si l'état de fertilité est moyen est sélectionnée dans le groupe consistant en les suivantes : insémination intracervicale (ICI), pré-capacitation du sperme avant insémination intracervicale, insémination intra-utérine (IUI) ou pré-capacitation du sperme avant insémination intra-utérine ; fécondation *in vitro* (FIV) ou pré-capacitation du sperme avant fécondation *in vitro* ; ou
l'approche reproductive utilisée si l'état de fertilité est faible est sélectionnée dans le groupe consistant en les suivantes : fécondation *in vitro* (FIV), pré-capacitation du sperme avant fécondation *in vitro,* injection intracytoplasmique de spermatozoïdes (ICSI), pré-capacitation du sperme avant injection intracytoplasmique de spermatozoïdes, transfert intratubaire de gamètes (GIFT), pré-capacitation du sperme avant transfert intratubaire de gamètes, injection subzonale de spermatozoïdes (SUZI) ou pré-capacitation du sperme avant injection subzonale de spermatozoïdes.
